(19) Europäisches Patentamt
European Patent Office
Office européen des brevets

(11) Publication number: **0 623 672 A1**

# (12) EUROPEAN PATENT APPLICATION

(21) Application number: **93107233.4**

(22) Date of filing: **04.05.93**

(51) Int. Cl.⁵: **C12N 9/58**, A01N 63/04, //(C12N9/58,C12R1:79)

The applicant has subsequently filed a sequence listing and declared, that it includes no new matter.

(43) Date of publication of application: **09.11.94 Bulletin 94/45**

(84) Designated Contracting States: **DE**

(71) Applicant: **RESEARCH INSTITUTE FOR PLANT PROTECTION**
**Binnenhaven 12,**
**P.O. Box 9060**
**NL-6700 GW Wageningen (NL)**

(72) Inventor: **Den Belder, E., Ir.**
**Reeboklaan 22**
**6705 DB Wageningen (NL)**
Inventor: **Bonants, P.J.M., Dr.**
**Candialaan 8**
**3911 BA Rhenen (NL)**
Inventor: **Fitters, P.F.L., Ir.**
**Mennonietenweg 23**
**6702 AB Wageningen (NL)**
Inventor: **Waalwijk, C., Dr.**
**van der Lecklaan 21**
**6708 MN Wageningen (NL)**

(74) Representative: **Schmidt, Werner, Dr.**
**Hoechst AG**
**Zentrale Patentabteilung**
**Postfach 80 03 20**
**D-65926 Frankfurt am Main 80 (DE)**

## (54) New alkaline serine protease of Paecilomyces lilacinus.

(57) The invention relates to a protease from Paecilomyces lilacinus, a process for the preparation of the protease, which comprises cultivation of Paecilomyces lilacinus and isolation of said protease. More generally the invention relates to the use of the protease for the controll of plant parasitic nematodes. The invention further relates to the gene for this protease.

EP 0 623 672 A1

1. Introduction

In many crops nematodes are important parasites. Chemical control of these parasites meet with growing objections because of environmental effects. Alternatives of these control measurements have to be developed. Biological control of nematodes seems to be a good alternative. Many fungi parasitize on plant parasitic nematodes, either by capturing nematodes or by parasitizing the nematode eggs.

Several fungi are capable to penetrate the eggs of nematodes (Bursnall & Tribe (1974), Stirling & Mankau (1979), Chalupova & Lenhart (1984), Kunert et al. (1987), Dackmann et al. (1989) and Gaspard et al. (1990). Those fungi are important objects for research concerning potential use as biological control agents of nematodes. Hyphae of Dactylella oviparasitica grow rapidly through egg-masses of the root-knot nematode Meloidogyne spp. and the fungus penetrated egg-shells (Stirling & Mankau (1979). Dackmann et al. (1989) investigated fungal egg-parasites isolated from eggs of the cyst nematode Heterodera avenae with respect to their ability to infact cyst nematode eggs of H. schachtii in vitro. Of these Verticillium suchlasporium appeared to be the most effective parasite. Gaspard et al. (1990) and de Leij & Kerry (1991) reported on Verticillium chlamydosporium as control agent for Meloidogyne spp. Also the nematode egg-parasitic fungus Paecilomyces lilacinus (Thom) Samson has been studied for its ability to control root-knot nematodes (Jatala et al. (1979, 1980), Dunn et al. (1982), Villanueva & Davide (1984), Davide & Batino (1985), Culbreath et a (1986), Dube & Smart (1987), Cabanillas & Barker (1989), Cabanillas et al. (1989), Gaspard et al. (1990).

The mechanism of the infection process of egg-parasitic fungi could be mechanical and/or enzymatic in nature. Little is known about the role of enzymes involved. To date few attempts have been made to determine the effects of purified fungal enzymes on the egg-shell of the root-knot nematode. Research on enzymes of nematode egg-parasitic fungi was done in liquid culture of Dactylella oviparasitica supplemented with colloidal chitin. Stirling & Mankau (1979) found chitinase activity and suggested a role for this enzyme in pentrating the egg-shell, which consists partly of chitin. If any other enzyme was involved was unclear. Dackmann et al. (1989) showed a correlation between infection of egg-parasites of cyst nematode eggs Heterodera schachtii in vitro and lytic enzyme activity.

Especially chitinase and protease activity seemed to be involved. Lopez-Llorca (1990) purified an extracellular protease from the fungal parasite of eggs of cyst nematodes, Verticillium suchlasporium, and proved that this protease was capable of degrading a protein from the egg-shell of cyst nematodes. Lopez-Llorca & Robertson (1992) immunolocalized cytochemically the presence of this protease in infected nematode eggs and supported therefore the role for this enzyme in the pathogenicity of the fungus to nematode eggs.

Den Belder et al. (1993) showed SEM pictures of root-knot nematode eggs infected by P. lilacinus suggesting clearly the involvement of enzymes.

The products of enzymes and especially proteases of several organisms, which are able to degrade a certain kind of shell, for example insect cuticles or fungal cell walls, were studied (Gabriel (1968), Leopoled & Samsinakova (1970), Samsinakova et al. (1971), Latgé (1974), Kucera (1980), Smith et al. (1981), St. Leger et al. (1986a, 1986b, 1986c, 1987a, 1991), Geremia et al. (1991). St. Leger and co-workers (1987a) showed that the proteases, produced in situ during pentration of Calliphora vomitoria and Manduca sexta cuticles by hyphae of the entomopathogenic fungus Metarhizium anisopliae, were the same as those produced in culture media. Production of the protease without differentation of infection structures of M. anisopliae can occur rapidly by nutrient deprivation alone (St. Leger et al. (1988). The proteases of two entomopathogenic fungi, M. anisopliae and Beauveria bassiana have been purified (St. Leger et al. (1986a, 1987b), Bidochka & Khachatourians (1987). Gold-labelled antiserum was used to demonstrate that a culticle-degrading protease (Pr1) is produced by M. anisopliae during penetration of host (Manduca sexta) procuticle (Goettel et al. (1989)).

The nematode egg-parasite Paecilomyces lilacinus, is able to degrade the egg-shell of the root-knot nematode Meloidogyne hapla (Dunn et al. (1982) and is used as a biological control agent in Peru (Jatala et al. (1980) and in the Philippines (Villanueva & Davide (1984). The egg-shell of the root-knot nematode M. hapla consits for at least 40 % of protein (Bird & McClure (1976), Bird (1976)). The outer layer of the egg-shell consits of vitellin, also a protein, and is therefore the first substrate for the fungus.

2. Description of the invention

The invention relates to

1. A alkaline serin protease from Paecilomyces lilacinus having
   - a molecular weight of 20,00 to 200,000 Dalton

- an isoelectric point at a pH between 9 and 12,
- a pH optimum in a range from 9 and 12 and
- a substrate specificity towards surface structures of plant parasitic nematodes.

2. A process for the preparation of the protease characterized under 1, which comprises cultivation of Paecilomyces lilacinus and isolation of said protease.

3. The use of the protease characterized under 1 for the controll of plant parasitic nematodes.

4. The invention further relates to the gene for a protease, having the DNA-sequence shown in the sequence protocoll 1 and for a gene coding for an enzyme having the same action and whose amino acid sequence is coded by the DNA sequence shown in sequence protocoll 1 and Figure 10 and derived from that sequence by addition, deletion or exchange of nucleotides.

The serin protease from Paecilomyces lilacinus exhibits a substrate specificity towards surface structures of plant parasitic nematodes, preferably the egg shell, especially vitellin.

The investigation revealed that the enzyme is astonishingly active at high temperatures. This this feature can be used to distinguish and separate the enzyme from other proteins by thermal denaturation.

The isolation and purification can be carried out as described in the materials and methods and in the examples.

The enzyme preparation can be characterized by a molecular weight of 20,000 to 200,000, preferably 25,000 to 100,000, especially 30,000 to 50,000 Dalton.

The enzyme can be characterized furthermore by an isoelectric point which is at a pH of 8 to 12, preferably 9 to 11, especially 9,5 to 12.

The pH optimum of the enzyme product is in the range of 7 to 12, preferably 8 to 11, especially 9,5 to 11.

The reaction temperature can range between 40 and 80 °C, preferably between 50 and 70, especially between 55 and 65 °C.

The protein sequence of 16 N-terminal amino acids have been determined. This part of the amino acid sequence shows homology with proteases previously disclosed in the literature.

It is possible according to the invention to use that transaminase directly or indirectly for the controll of plant parasitic nematodes.

The invention additionally relates to plasmids containing a gene of this type, and to microorganisms, in particular E.coli and fungi, containing a plasmid of this type.

Another object of the invention is to provide a new process for controlling the action of nematodes on plants and plant cells. The gene can be used to controll plant parasitic cyst, root-knot and lesion nematodes, especially root-knot nematodes. In a preferred embodiment the gene is used to controll the growth of Meloidogyne hapla and Meloidogyne incognita belonging to the Heteroderidae. This family includes also the potato cyst nematode and the sugar beet cyst nematode.

Another object is to provide DNA fragments which comprise DNA sequences capable of protecting plants and plant cells, when incorporated and expressed therein against the action of plant parasitic nematodes.

Plants which can be protected are either monocotyledons or dicotyledons.

Examples of families that are of special interest are Solanaceae and Brassicaceae. Examples of species of commercial interest that can be protected include:

| | |
|---|---|
| - tobacco, Nicotiana tabacum L.<br>- potato, Solanum tuberosum L.,<br>- Canola/Rapeseed,<br>- cabbage, broccoli, kale etc.,<br>- mustards Brassica juncea L.,<br>- Brassica nigra L., and Sinapis alba L. (Brassicaceae),<br>- sugar beet, Beta vulgaris, (Chenopodiaceae),<br>- cucumber, Curcurbita sp. (Curcurbitaceae),<br>- cotton, Gossypium sp., (Malvaceae),<br>- sunflower, Helianthus annuus,<br>- lettuce Lactuca sativa, (Asteraceae = Compositae),<br>- pea, Pisum sativum,<br>- soybean, Glycine max and alfalfa, Medicago sp. (Fabaceae = Leguminoseae),<br>- asparagus, Asparagus officinalis;<br>- corn, Zea mays and | - tomato, Lycopersicon esculentum Mill,<br>- petunia, Petunia hybrida (Solanaceae)<br>- Brassica napus L.,<br>- Brassica oleracea L.,<br>- gladiolus, Gladiolus sp., (Lilaceae);<br>- rice, Oryza sativa (Poaceae). |

In an preferred embodiment the gene prevents diseases of plants such as potato, tomato, wheat, cabbage and chinese cabbage.

3. Material & Methods

The invention is desribed in detail hereinafter, especially in its preferred embodiment by way of examples for non-limitive illustration purposes. The invention is furthermore defined in the claims.

3.1 Culture of fungus

Paecilomyces lilacinus (Thom.) Samson (CBS 143.75), obtained from the CBS (Central Bureau of Fungal Cultures) in Baarn (The Netherlands), was routinely maintained (once a month) on Potato Dextrose Agar (PDA; difco laboratries) in 90 mm petridishes at 25 °C. A conidial suspension was obtained by adding 5 ml of sterilized water to a PDA petridishes containing sporulating mycelium and scraping the surface with a glass rod. Liquid cultures were obtained by inoculating conidia of the fungus to minimal salt medium or corn flour medium supplemented with the substrate. The minimal salt medijm (MM) consisted of 4.56 gr $K_2HPO_4$, 2.77 gr $KH_2HPO_4$, 0.5 gr $MgSO_4 . 7H_2O$ and 0.5 gr KCl / liter pH 6.0.

The corn flour medium (CFM) was prepared by steaping 40 gr of grinded corn flour in 1 l demineralized water for 1 hour at 55 °C. Next the mixture was filtered over Whatman no 1 filter and the filtrate was used after autoclavation (20 minutes 120 °C).

The following substrates were added alone or in combination when required: vitellin (0.2 % (w/v); Sigma), collodial chitine (0.2 % and 1.0 % (w/v); prepared according to Lingappa & Lockwood (1962) using chitin from Sigma), root-knot nematode eggs ( > 400.000; isolated as described below), nitrogen (N) (as 2.0 gr asparagine + 2.0 gr $NaNO_3$/l) and glucose (G) (2.0 % (w/v).

In one experiment mycelium of Paecilomyces lilacinus was used as inoculum. Mycelium was obtained by centrifuging a 6 day old culture of conidia of P. lilacinus in MMNG for 45 minutes at 9000 g. Cultures were grown in a shaking waterbath for several days at 30 °C and 125 strokes per minute. Culture filtrates were obtained by centrifuging cultures for 45 min at 9000 g. Supernatants were concentrated using an Amicon (YM-10 filter) and clarified through a 0.22 µm-filter (Schleicher & Schuell). Pellets were freeze dried and the weigth of the mycelium was measured.

To obtain culture filtrates for daily testing of protease activity proteins content and glucose concentration 1 ml aliquots were removed from the culture and centrifuged for 15 minutes at 14.000 g. The resulting supernatant was clarified through a 0.22 µm-filter (Schleicher & Schuell).

Escherichia coli cells were grown in LB broth (1 % bacto tryptone, 0.5 % yeast extract, 0.5 % Sodium chloride), when necessary 100 µg/ml ampicillin was added. E. coli strain PLK-F'(mrcA-, mrcB-, recA-, $Tet^R$) was used for the preparation of plating cells. E. coli strain InVaF' competent cells were purchased from Invitrogen (San Diego) and used as carrier of recombinant plasmids.

A spore suspension of Paecilomyces lilacinus was obtained by adding 5 ml of sterilized water to a PDA plate containing sporulating P. lilacinus mycelium and scraping the surface with a glass rod. Liquid cultures for genomic DNA isolations were grown in potato dextrose broth (PDB; Difco). The liquid induction medium (IM) used for the isolation of the mRNA fraction contained 0.5 gr/L KCl, 0.5 gr/L $MgSO_4$, 1.36 gr/L $KH_2PO_4$, 2.28 gr/L $K_2HPO_4$ and $4x10^8$ nematode eggs/L as the sole carbon and nitrogen source. As an inoculum $4x10^{10}$ spores/L was used.

3.2 Culture of nematode

The root-knot nematode Meloidogyne hapla Chitwood, obtained from the Centre for Plant Breeding and Reprodcution Research (CPRO-DLO) in Wageningen, was continiously maintained in a greenhouse on tomato plants (Lyopersicom esculentum cv. Moneymaker) for 8 hr at 15 °C and 16 hr at 20 °C per day with a relative humidity of ≧ 65%. Artificial lighting was supplied to give 16 hours daylength. The plants are grown in sterilised silver sand and nutrients were supplied according to Steiner.

The Northern root-knot Meloidogyne hapla was reared on tomato plants (lycopersion esculentum cv. Moneymaker) in a greenhouse. Six week-old plantlets were inoculated by placing 2000 nematode eggs in the vicinity of the stem. Eight to ten weeks after inoculation, nematode eggs were collected by vigorously shaking the nematode-infected roots in 1 % (v/w) hypochloride for 10 min. The liberated eggs were collected by centrifugation ($2x10^3$ rpm, 10 min), and loaded onto a continous sucrose gradient, prepared by freezing and slowly thawing a 40 % (w/v) sucrose solution. The gradient was spun ($2x10^3$ rpm, 10 min) and the eggs were collected, washed several times with sterilized, distilled water, and counted.

3.3 Isolation of nematode-eggs.

Eggs of the root-knot nematode M. hapla were isolated according to Schaad & Walker (1975). Roots of tomato plants infected with the root-knot nematode M. hapla for more than 6 weeks were rinsed with water to remove the soil. Then they cut into small parts and shaken with 1.0 % hypochlorite for 5-10 minutes. Eggs were collected from the solution by sequential passage over 200,75 and 22 µ sieves (Retsch (200 and 75 µ) and Scrynel (22 µ) and separated plant material through sucrose gradient centrifugation (0-30 % sucrose and 5 minutes centrifugation at 1600 g in sterile 50 ml Greiner tubes). Ultimately eggs (on top of the gradient) were washed several times with sterile water.

3.4 Protease activity

Protease activity was determined by a modified procedure of Rinderknecht et al. (1968). Ten mg of Hide Powder Azure (Sigma) was incubated in 50 mM Sodium acetate buffer pH 6.0 with the sample in a final volume of 3 ml in small glass containers. Incubation was at 37 °C in a shaking waterbath (110 strokes-(min) till the solution turned blue (between 5 and 30 minutes). Following incubation the samples were put on ice and centrifuged in 1.5 ml Eppendorf cups for 5 minutes at 14.000 g. The absorbance of the supernatant was measured at 595 nm on a Uvikon 940 spectrophotometer. Protease activity of the sample was measured in duplo, averaged and expressed as $A_{595}$ $ml^{-1}hr^{-1}$ after correction for the blank (Milli Q water).

Protease activity on gelatine agar plates was determined by incubating conidia of P. lilacinus on 1.2 % (w/v) NM agar at 25 °C containing 0.2 % (w/v) gelatin. Halo's indicating extracellular proteolytic activity were visualized by staining with Coomassie Brilliant Blue.

Protein quantification of the samples wer determined according the Bradford (1976), using BSA (bovine serum albumine) as the standard.

Concentration of D-glucose of the samples were determined with the enzymatic UV-method of Boehringer (Cat.no. 139106).

SDS-PAGE was performed by the method of Laemmli (1970). Culture filtrate samples were precipitated with trichloroacetaic acid, washed with ice-cold aceton, dried, suspended in sample-buffer (62.5 nM Tris/HCl pH 6.8, 10 % glycerol, 2 % SDS, 10 nM DTT, 0.1 % BPB) and boiled for 5 minutes. Native PAGE in the presence of gelatin was performed according to Heussen % Dowdle (1980).

The concentrated culture filtrate was dialyzed overnight at 4 °C against equilibration buffer (100 nM Ammoniumacetate, 10 mM $CaCl_2$, pH 6.5) and applied to an equilibrated 2 ml Bacitracin-Sepharose column. The Bacitracine-Sepharose was made as described by Stepanov & Rudenskaya (1983). After washing the column with 100 mM Ammoniumacetate buffer pH 6.5, the bound protease was eluted with elution buffer (100 mM Ammoniumaceatete, 1 M NaCl, 25 % isopropanol pH 6.5). Fractions of 1 ml were collected and tested for proteolytic activity. Positive fractions were pooled and dialyzed agianst 100 nM Ammonium acetate pH 6.5.

3.5 Characterization of the protease

- Molecular weigth

After SDS-Page gels were stained with silver (Morrissey (1981)) and dried with the Kem-en Tec gel drying frame. Densitograms of the gels were obtained with the video densitometer Model 620 of BioRad. Molecular weight was determined using BioRad's Low Molecular Weight standards as reference (rabbit muscle phosphorylase b (97,400 Da), bovine serum albumine (66,200 Da), hen egg white ovalbumine (45,000 Da), bovine carbonic anhydrase (31,000 Da), soybean trypsin inhibitor (21,500 Da) and hen egg white lysozyme (14,400 Da)).

- Optimum pH

For the determination of the optimal pH for protease activity the following buffers were used:

| | |
|---|---|
| pH 4.0 - 5.7 | Sodium acetate |
| pH 6.0 - 7.6 | Potassium phosphate |
| pH 7.7 - 9.0 | Tris/HCl |
| pH 9.2 - 10.6 | 2-Amino methyl propanol |
| pH 10.6 - 12.6 | 2-Amino methyl propanol |

Protease activity was performed as described above in triplicate.

- Optimum temperature

For the determination of the optimum temperature for protease activity the proteolytic activity measurements were performed as described above in triplicate at different temperatures.

- Isoelectric point

For the determination of the isoelectric point the protease was applied to a Mono P chromatofocusing column (HR 5/5 from Pharmacia) at pH 10.2 (25 mM 2-amino-2-methyl-1-propanol/HCl). With an FPLC (Fast Protein Liquid Chromatography) system gradient elution was performed with Polybuffer (1:10) pH 8.0.

Iso-electrofocusing (IEF) was employed with the Phastsystem of Pharmacia using IEF-gels with a pH-gradient from 3 to 10 according to the instructions of the manufactory.

- Protease inhibition

Several protease inhibitors, SH reducing agents and metal ions were tested on the proteolytic activity of the protease: PMSF, E64, 1.10-phenantroline, pepstatine (Sigma); EDTA, cystein $MgCl_2$ and $CaCl_2$ (Merck); DTT (BioRad). The purified enzyme was incubated for 40 minutes at 4 °C with inhibitor. After incubation the protease-activity was determined as previously described. Final inhibitor concentration was 1 mM. Protease activity in the absence of inhibitor was expressed as 100 %.

- Binding of protease to root knot nematode eggs

Root-knot nematode eggs were incubated at room temperature with the purified protease in 1.5 ml Eppendorf cups in 100 mM Potassium phosphate buffer pH 7.0 with continously shaking.

On several time points 200 µl samples were taken, centrifuged for 1 minute at 14.000 g. Protease activity and SDS-PAGE was performed on the supernatant. The bound enzyme was eluted from the nematode eggs with 100 mM Potassium phospate buffer containing 0.5 M NaCl.

- Degradation of vitellin by the serine protease

Vitellin (1 mg) was incubated with the purified protease for 16 hours at 37 °C in 1.5 ml Eppendorf cups in 100 mM Sodiumacetate buffer pH 6.0 (final volume was 1 ml). After incubation the samples were centrifuged (1 minute 14000 g) and absorption of the supernatant was mesaured at 280 nm ona Uvikon 940 spectrophotometer.

3.7 Recombinant DNA techniques.

All restriction enzymes and T4 ligase were obtained from Gibco BRL and used as recommended by the supplier. Methods were performed as described in Maniatis et al. (1982).

3.8 Isolation of genomic DNA from P. lilacinus

P. lilacinus was grown for 2-4 days in PDB at 30 °C and 150 rpm. The mycelium was collected by filtration through myracloth (Calbiochem Corporation, La Jolla). The mycelium was frozen in liquid nitrogen and grinded to a fine powder in a mortar. Per gram powder, 5 ml of extraction buffer (0.1 M NaCl, 10 mM Tris-HCl (pH 7.5), 1 mM EDTA, 1 % SDS) was added and the suspension was vortexted for 5 min. An equal volume of phenol/chloroform (1:1) was added and the phases were vigourously mixed. After centrifugation (20 min, $10^4$ rpm), 0.1 volumes of 3 M NaAc (pH 5.2) and 2 volumens of ethanol were added to the aqueous phase. After precipitation (30 min, -20 °C), the nucleic acid fraction was pelleted by centrifugation (20 min, $10^4$ rpm). The pellet was resuspended in 2 ml of water. RNase was added to a final concentration of 10 µg/ml and the solution was incubated at 37 °C for 30 min. The solution was extracted once with phenol/chlorform (1:1) extraction, once with chloroform and precipitated with ethanol.

3.9 Isolation of mRNA from P. lilacinus

P. lilacinus was grown for 48 hours in IM at 30 °C and 150 rpm. The mycelium was collected by filtration through myracloth. The mycelium was frozen in liquid nitrogen and grinded to a fine powder in a mortar. Per gram powder, 5 ml of extraction buffer (0.1 M NaCl, 10 mM Tris-HCl (pH 7.5), 1 mM EDTA, 1 % SDS) was added and the suspension was vortexted for 5 min. An equal volume of phenol/chloroform (1:1) was added and the phases were vigourously mixed. After centrifugation (20 min, $10^4$ rpm), 0.1 volumes of 3 M NaAc (pH 5.2) and 2 volumes of ethanol were added to the aqueous phase. After precipitation (30 min, -20 °C), the nucleic acid fraction was pelleted by centrifugation (20 min, $10^4$ rpm). The pellet was resuspended in sterile, distilled water and the mRNA fraction was isolated, using the polyATract system (Promega, Madison). Approximately 15 µg of mRNA was obtained from 200 ml culture. Using the lambda ZAP system (Stratagene, La Jolla) a cDNA library was contructed starting with 5 µg mRNA. The library was found to contain 500.000 individual clones.

3.10 Polymerase chain reaction

The PCR reaction mixture of 100 µL contained 50 mM KCl, 10 mM Tric-HCl (pH 8.3), 0.5-2 mM $MgCl_2$, 100 µM of each dNTP, 100 pmol of oligonucleotide A and oligonucleotide B (see below), 400 ng of genomic DNA from P. lilacinus, and 2.5 units of AmpliTaq DNA polymerase (Perkin Elmer mineral oil. Each of the 35 amplification cycles included a denaturation step at 94 °C for 1 min, an annealing step at 42 °C to 60 °C for 2 min, and a chain elongation step at 72 °C for 3 min. The amplification reaction was preceded by a denaturation step at 94 ° for 0.5 min, and the elongation step of the last cycle was extended to 5 min.

3.11 Screening of the cDNA library

The library was plated at a density of 10.000 plaques/plate on PLK-F' cells. After overnight incubation at 37 °C, duplicate nitrocellulose filters or each plate were prepared according to Maniatis et al (15). The filters were baked at 80 °C for 2 hours and incubated in prehybridisation solution (6x SSC, 5x Denhardt's solution, 0.1 % SDS, 100 µg/mL denatured salmon sperm DNA) at 65 °C for 4 hours.

To obtain a radioactive probe, 1 µL of the PCR reaction product was used. The labelling reaction mixture was identical to the PCR reaction mixture, except that the dATP ws substituted for 5 µL alpha-$^{32}$P-dATP (3000 Ci/mmol, 10 mCi/ml). To incorporate the radioactive nucleotides, 7 amplifiaction cycles were performed identical to the cycling conditions used to obtain the DNA fragment.

The probe was separated from the free nucleotides using a Sephahdex G-50 spin column. After denaturation (10 min, 100 °C), the probe was added to prehybridisation solution and the filters were hybridized for 16 hours. The filters were washed twice 2x SSC/0.1 SDS for 30 min at 65 °C, and once with 0.2x SSC/0.1 SDS for 30 min at 65 °C. X-ray film (Fuji RX) was exposed to the filters for 16 hours and a autoradiograph was obtained. Positives were rescreened by the same procedure until pure. After in vivo excision of the plasmids according to the manual supplied by the manufacturer (Stratagene), they were subjected to restriction enzyme analysis.

3.12 Sequence analysis

The insert of pSP3 was partially sequenced using the Taq dye primer cycle sequencing kit (Applied Biosystems, Foster City) and an Biorad R370 automated sequencer.

4. Examples

The nematode egg-parasitic fungus Paecilomyces lilacinus penetrates the egg-shell of the root-knot nematode Meloidogyne hapla which consits for a great part of proteins. In the following induction, purification and characterization of this protease is described.

4.1 Induction protease

Paecilomyces lilacinus grown for 3 days at 25 °C on solid agar containing gelatin showed halo's after staining with Coomassie Brilliant Blue proving extracellular production of proteases. Subseqently the production of extracellular protease by the fungus in liquid minimal salt medium (MM) and in liquid corn flour medium (CFM), to which several substrates were added, was studied. Protease activity of the culture filtrate was monitored at daily intervals following inoculation of the medium with conidia of the fungus. Figure 1 shows the protease activities of the culture filtrate with eggs (E) as substrate and with nitrogen and glucose (NG) as control on several days after inoculation. Even after two days protease activity was increased in CFM cultures containing eggs (CFME). It reaches maximum at 3 days after inoculation and decreases to zero protease activity on day 7. On day 3 MM cultures containing eggs (MME) showed also a substantial increase in proteolytic activity. On day 10 activity was still increasing. MM supplemented with nitrogen and glucose (MNNG) showed an increase of protease activity similar to MME. Addition of chitin (C) or vitellin (V) as substrate to cultures of P. lilacinus in MM resulted also in induction of proteolytic activity. Table 1 shows protease activity and protein content of the filtrate of 4 different cultures of P. lilacinus after 4 days growth. The highest induction of protease activity was seen with vitellin as substrate. Specific protease activity (protease activity/ µg protein) was highest in cultures containing eggs as substrate.

To see whether the fungus used glucose as carbon sourece, glucose concentrations were determined in the culture filtrate, of the fungus in MNNG (Figure 1). After day 4 almost all glucose was used. Glucose is a well known repressor of the induction of many enzymes. To study if catabolite repression was involved in the induction of P. lilacinus protease, glucose was added at a concentration of 0.3 % each day to MNNC. In this experiment mycelium of P. lilacinus was used as inoculum. Figure 2 shows that the induction of protease activity was repressed by glucose. Adding conidia or mycelium (harvested after incubating the same amount of conidia of P. lilacinus as inoculum in MNNG for 6 days) resulted in no differences in induction of the proteolytic activity.

To determine the influence of nitrogen in the medium on the induction of protease nitrogen was added to MMGC or to MMGE. The results after 4 days of growth in Table 2. When there is no nitrogen and no substrate present in the medium (MMG) no proteolytic activity could be detected in the culture filtrate and no glucose had been consumed. There was no growth of the fungus. In the presence of substrate (C, E) protease activity was observed and glucose had been used. In the culture with chitin as substrate (MMGC) glucose concentration decreased to 50 %, with eggs as substrate (MMGE) it decreased to 90 %. When nitrogen was present in the medium and no substrate (MNNG), glucose concentration lowered to 15 % with low protease activity. Addition of eggs as substrate (MNNGE) resulted in high protease activity and complete glucose consumption. Chitin as substrate (MNNGC) resulted in low protease activity and also complete glucose comsumption. The weigth of mycelium was highest in MNNGC. Specific protease activity (protease activity per mg mycelium) was highest in MMGE.

4.2 Purification of protease

The SDS-PAGE patterns of the culture filtrates showed many proteins produced in the different cultures (Figure 3). The protein pattern of the culture filtrate with vitellin as substrate looked very similar to the one of the culture filtrate with nematode eggs as substrate (lane 3 and 5 respectively).

To identify the nature of the protease the proteolytic activity in the culture filtrate was inhibited with several protease inhibitors. Inhibition of the proteolytic activity in the culture filtrate with PMSF (a serine protease inhibitor) showed that most of the protease activity present in the medium was inhibited suggesting a serine protease being involved as the most secreted protease. Purification of the serine protease(s) was first performed with Benzamidine-Sepharose, an affinity resin for serine proteases of class I (Pharmacia). Since all of the proteolytic activity did not bind, the protease belonged to another serine protease class, the subtilisin class. Stepanov & Rudenskaya (1983) showed that this class of proteases binds to Bacitracin-Sepharose.

Figure 4a shows the proteolytic activity of fractions of the Bacitracin-Sepharose column to which concentrated culture filtrate of the fungus (grown for 4 days in MM with chitin a substrate) was applied. After washing and elution the protease activity was predominantly found in fractions 8 and 9. Figure 4b shows the SDS-PAGE patterns of fractions of the affinity purification step. Integration of the densitogram of fraction 8 revealed that one protein was present for more than 85 %. It was concluded that this was the serine protease. The same protein was purified from culture filtrates of P. lilacinus grown with eggs and vittelin as substrate. All substrates induced the serine protease.

4.3 Characterization of protease

The molecular weight of the serine protease of Paecilomyces lilacinus using molecular weigth markers was 33.5 kDa.

Since the protease did not bind to the Mono P column at pH 10.2 the isolectric piont should even be higher. Isoelectric focussing using the Phastsystem of Pharmacia and pH 3-10 gels showed that the protease focussed at pH 10.

The optimum pH and temperature for proteolytic activity was obtained by performing protease activity measurements at different pH and temperature respectively. Figure 5 shows an optimum pH of 10.3 after fitting of the protease activity curve. Figure 6 shows the temperature activity profile for the P. lilacinus protease. Optimum temperature for the proteolytic activity was about 60 °C.

In order to elucidate the type of protease involved a series of inhibitors, SH-reducing agents and some metal ions were tested on the proteolytic activity of the protease. Figure 7 shows the effect of several protease inhibitors, some SH-reducing agents and some metal ions on the activity of the purfied protease. PMSF inhibited the activity for 100 %. Consequently the protease is of the serine protease class. All other protease inhibitors tested did not effect the proteolytic activity of the enzyme significantly. DTT and cystein did not influence its activity also suggesting no SH-groups being involved in the active center of the enzyme. $Ca^{++}$- and $Mg^{++}$-ions lowered the protease activity slightly. EDTA on the other hand enhanced its activity suggesting a negative influence of metal ions present in the assay.

The purified protease was capable of degrading insoluble vitellin and produced halo's on MM agar plates containing gelatin.

Incubation of the protease with nematode eggs at room temperature showed that the protease bound quantitatively to the eggs shown by activity measurements on the supernatant and that the eggs floated after overnight incubation. Control eggs did not. The bound protease could be eluted from the eggs by 0.5 M CaCl as shown by activity measurements and SDS-PAGE.

4.4 Design of oligonucleotides

The method used for the isolation of cDNA clones encoding the P. lilacinus serin protease (Psp) was based on the amplification of a part of the gene by the polymerase chain reaction. The design of the set of oligonucleotides used in the PCR reactions, was based on both amino acid sequence data on the Psp protein and on amino acid sequences which are conserved among subtilisin-like proteases.

| | Panel A. | Panel B. |
|---|---|---|
| A | GLTTQKSAPWGLSIS | DSIGHGTHVSGT |
| B | EFDTQNSAPWGIARIS | DGNGHGTHCAGT |
| C | AIQTTPVTQWGLSRIS | DLLGHTHVAGT |
| D | AAQTNAPWGLARIS | DGNGHGTHCAGT |
| | XXX XX | X XXXXX XX |
| E | AYTQQPGAPWGLGRIS | DGNGHGTHCAGT |

Comparison of the N-terminal sequence of Psp to the N-termini of other subtilisin-like protease. A, Aspergillus oryzae Alp (Tarsumi et al.); B, Saccharomyces cerevisiae protease B (MOEHLE et al.); C, Yarrowia lipolytica alkaline protease (Davidow); D, Tritirachium album, proteinase K (JANY et al.); E, Paecilomyces lilacinus Psp. Conserved residues are indicated by (x).

Panel B: Comparison of the amino acid sequences surrounding the active site His residue (indicated by *). A-D, as in panel A; E, hypthetical sequence use for the design of oligonucleotide B. F: Graphical representation of the position of the oligonucleotides used.

The 16 amino acids of the N-terminus of Psp were determined to be Ala-Tyr-Thr-Gln-Gln-Pro-Gly-Ala-Pro-(His?/Cys?/Trp?)-Gly-Leu-Gly-Arg-Ile-(Ser). Comparison of this sequence to other amino acid sequences of fungal subtilisin proteases, as compiled by Tatsumi et al., showed that the C-terminal part of the 15-amino acid stretch contained a conserved region, whereas the N-terminal part showed little homology (figure 8). The first oligonucleotide was based on this stretch of 9 amino acids to avoid cross-reactions with other subtilisin-like proteases that P. lilacinus may produce.

Matrix comparison of amino acid sequences of proteases of the subtilisin family reveals several highly conserved regions, including the regions surrounding the three residues (Asp 32, His 64 and Ser 221 in subtilisin BPN) composing the catalitic triad. The second oligonucleotide used in this study was based on the conserved region surrounding the active site histidine residue (figure 8). The distance between the N-terminus and the active site His-residue is 69-77 amino acids in fungal subtilisins (Davidow et al., Jany et al., Moehle et al., Tatsumi et al.).

To avoid strong degeneration of the oligonucleotides, inosine residues were used where four-base wobble occured. Degeneracy was allowed at sites where two-base wobble occured.

Primer A: 5' GCITAYACICARCARCCIGGIGCICC 3'
Primer B: 5' GTICCIGCRCARRGIGTICCRTGICCRTTICC 3'

Sequence of primer A and primer B. I, inosine residue; Y, C or T; R, A or G (see also Figure 8).

4.5 Polymerase chain reactions

To obtain a single reaction product, the temperature of the annealing step of the cycles was varied from 42 °C to 60 °C, resulting in multiple bands in all experiments. The annealing temperature was set at 60 °C and the $MgCl_2$-concentration was optimized. Using a $MgCl_2$-concentration of 0.5 mM, a single DNA band was seen on an agarose gel. The size of the band was estimated to be 240 basepairs. Although several other bands were seen at higher $MgCl_2$-concentrations, the 240-bp band was always the most predominant. This is approximately the expected size, assuming that the distance between N-terminus and the active site histidine residue in Psp is similar to that of other fungal subtilisin-like proteases (69-77 amino acid residues).

4.6 Screening of cDNA library.

The PCR reaction product containing the single visible 240-bp DNA band was used a radioactive labelled probe and hybridised to duplicate nitrocellulose filters containing 100.000 plaques in total. The resulting autoradiographs showed both strong and weak hybridisation signals. Seven strongly hybridizing plaques were identified and subsequently purified. Using the in vivo excision system offered by the lambda ZAP system, the 7 lambda clones were converted to plasmids.

4.7 Restriction enzyme analysis

A restriction map was made of the 7 plasmids, and based on these data they could be classified in two categories. The first category, consisting of 4 plasmids, containing an insert of 1400 base pairs. One of these plasmids, which was used in further studies, was named pSP3. The second category, consisting of 3 plasmids, contained an insert of 1200 bp. One of these plasmids was named pSP4. Based on restriction patterns, we concluded that the insert of pSP4 is a shorter version of pSP3 (figure 9).

To facilitate sequencing of the complete insert of pSP3, a subclone, pSP3-2, was created by digesting pSP3 with SmaI and religate the larger fragment containing the vector and 0.5 kb of the insert. +

4.8 Sequencing

pSP3 was partially sequenced. It was possible to identify the region containing oligonucleotide A (figure 9). This region is located 250 bp downstream of the EcoRI cloning site. Furthermore, a 3'poly(A) sequence was found.

The region just downstream of oligonucleotide A is reasonably consistent with the known N-terminal amino acid sequence of Psp, which leads us to believe that pSP3 encodes the Psp protease of P. lilacinus. The length of the insert of pSP3 upstream of the oligonucleotide A sequence suggests that this is a full length or nearly full length cDNA.

6. References.


BIDOCHKA, M.J. & KHACHATOURIANS, G.G. (1987). Purification and properties of an extracellular protease produced by the entomopathogenic fungus Beauveria bassiana. Applied and Environmental Microbiology 53, 7, 1679-1684.

BIDOCHKA, M.J. & KHACHATOURIANS, G.G. (1988). Regulation of extracellular protease in the entomopathogenic fungus Beauveria bassiana. Experimental Mycology 12, 161-168.

BIRD, A.F. & MC CLURE, M.A. (1976). The tylenchid (Nematode) egg shell: structure, composition and permeability. Parasitology 72, 19-28.

BIRD, A.F. (1976). The development and organization of skeletal structures in nematodes. In: The Organization of Nematodes, pp. 107-137. Edited by N.A. Croll. New York: Academic Press.

BRADFORD, M.M. (1976). A rapid and sensitive method for the quantitation of microgram quantities of protein utilizing the principle of protein-dye binding. Analytical Biochemistry 72, 248-254.

BURSNALL, L.A. & TRIBE, H.T. (1974). Fungal parasitism in cysts of Heterodera II. Egg parasites of H. schachtii. Transactions of the British Mycological Society 62, 595-601.

CABANILLAS, E. & BARKER, K.R. (1989). Impact of Paecilomyces lilacinus inoculum level and application time on control of Meloidogyne incognita on tomato. Journal of Nematology 21, 115-120.

CABANILLAS, E., BARKER, K.R. & NELSON, L.A. (1989). Growth of isolates of Paecilomyces lilacinus and their efficacy in biocontrol of Meloidogyne incognita on tomato. Journal of Nematology 21, 164-172.

CHALUPOVA, V. & LENHART, IK. (1984). The relation between proteolytic activity and ovicidal activity of mutants of Verticillium chlamydosporium. Acta Universitatis Palacki Olomuc (Olumouc) Facultas Medicina 106, 79-87.

CULBREATH, A.K., RODRIGUEZ-KABANA, R. & MORGAN-JONES, G. (1986). Chitin and Paecilomyces lilacinus for control, of Meloidogyne arenaria. Nematropica 16, 153-166.

DACKMAN, C., CHET, I. & NORDBRING-HERTZ, B. (1989). Fungal parasitism of the cyst nematode Heterodera schachtii: Infection and enzymatic activity. Microbiology Ecology 62, 210-208.

DAVIDE, R.G. & BATINO, E. (1985). Biological control of root-knot nematodes on cotton through the use of fungi Paecilomyces lilacinus (Thom) Samson and Gliocladium roseium Bainier as seed treatment. Philippine Agriculturist 68, 159-167.

DAVIDOW, L.S., M.M. O'Donnel, F.S. Kaczmarek, D.A. Pereira, J.R., DeZeeuw, and A.E. Franke (1987). Cloning and sequencing of the alkaline extracellular protease gene of Yarrowia lipolytica. J. Bacteriol. 169: 4621-4629.

DEAN, D.D. & DOMNAS, A.J. (1983). The extracellular proteolytic enzymes of the mosquito-parasitizing fungus Lagenidum giganteum. Experimental Mycology 7, 31-39.

DOMSCH, K.H., W. GAMS, and T.H. ANDERSON (19080). Compendium of soil fungi, volume I. Academic press, London.

DUBE, B. & SMART Jr., G.C. (1987). Biological control of Meloidogyne incognita by Paecilomyces lilacinus and Pasteuria penetrans. Journal of Nematology 19, 222-227.

DUNN, M.T., SAYRE, R.M., CARRELL, A. & WERGIN, W.P. (1982). Colonization of nematode eggs by Paecilomyces lilacinus (Thom) Samson as observed with Scanning Electron Microscope. Scanning Electron Microscopy III, 1351-1357.

GABRIEL, B.P. (1986). Enzymatic activities of some entomophtorous fungi. Journal of Invertebrate Pathology 11, 70-81.

GASPARD, J.T., JAFFEE, B.A. & FERRIS, H. (1980). Meloidogyne incognita survival in soil infested with Paecilomyces lilacinus and Verticillium chlamydosporium. Journal of Nematology 22, 176-181.

GEREMIA, R., JACOBS, D., GOLDMAN, G.H., MONTAGU VAN, M & HERRERA-ESTRELLA, A. (1991). Induction and secretion of hydrolytic enzymes by the biocontrol agent Trichoderma harzianum. In: Biotic Interactions and Soil-borne Diseases. pp. 181-186. Edited by A.B.R. Beemster, G.J. Bollen, M. Gerlach, M.A. Ruissen, B. Schippers & A., Tempel. Amsterdam: Elsevier.

GOETTEL, M.s., ST. LEGER, R.J. RIZZO, N.W., STAPLES, R.C. & ROBERTS, D.W. (1989). Ultrastructural localization of a cuticledegrading protease produced by the entomopathogenic fungus Metarhizium anisopliae during penetration of host (Manduca sexta) cuticle. Journal of General Microbiology 135, 2233-2239.

HELLMICH, S. & SCHAUTZ, K. (1988). Production of extracellular alkaline and neutral proteases of Ustilago maydis. Experimental Mycology 12, 223-232.

HEUSSEN, C. & DOWDLE, E.B. (1980). Electrophoretic analysis of plasminogen activators in polyacrylamide gels containing sodium dodecyl sulphate and copolymerized substrates. Analytical Biochemistry 102, 196-202.

JANY, K.D., G. LEDERER, and B. MAYER (1986). Amino acid sequence of proteinase K from the mold Tritirachium album Limber. FEBS Lett. 199: 139-144.

JATALA, P., KALTENBACH, R. & BOCANGEL, M. (1979), Biological control of Meloidogyne incognita acrita and Globodera pallida on potatoes. Journal of Nematology 11, 303.

JATALA, P. KALTENBACH, R., BOCANGEL, M., DEVAUX, J. & CAMPOS, R. (1980). Field application of Paecilomyces lilacinus for controlling Meloidogyne incognita on potatoes. Journal of Nematology 12, 226.

KUCERA, M. (1980). Proteases from the fungus Metarhizium anisopliae toxic for Galleria mellonella larvae. Journal of Invertebrate Pathology 35, 304-310.

KUCERA, M. (1981). The production of toxic protease by the entomopathogenous fungus Metarhizium anisopliae in submerged culture. Journal of Invertebrate Pathology 38, 33-38.

KUNERT, J. ZEMEK, J., AUGUSTIN, J. & KUNIAK, L. (1987). Proteolytic activity of ovicidial soil fungi. Biologia (Bratislava) 42, 7, 695-705.

LAEMMLI, U.K. (1970). Cleavage of structural proteins during the assembly of the head of bacteriophage T4. Nature (London) 227, 680-685.

LATGÉ, J.P. (1974). Activités protéolytique et chitinolytique de Cordyceps militaris. Entomophaga 19, 41-53.

DE LEIJ, F.A.A.M. & KERRY, B.R. (1991). The nematophagous fungus Verticillium chlamydosporium as a potential biological control agent for Melidogyne arenaria. Revue de Nematologie 14, 157-164.

LEOPOLD, M. & SAMSINAKOVA, A. (1970). Quantitative estimation of chintinase and other enzymes in the fungus Beauveria bassiana. Journal of Invertebrate Pathology 15, 34-42.

LINGAPAPPA, Y. & LOCKWOOD, J.L. (1962). Chitin media for selective isolation and culture of Actinomycetes. Phythopthalogy 52, 317-323.

LOPEZ-LLORCA, L.V. (1990). Purification and properties of extracellular proteases produced by the nematophagous fungus Verticillium suchlasporium. Canadian Journal of Microbiology 36, 530-537.

LOPEZ-LLORCA, L.V. & ROBERTSON, W.M. (1992). Immunocytochemical localization of a 32-kDa protease from the nematophagous fungus Verticillium suchlasporium in infected nematode eggs. Experimental mycology 16, 261-267.

MANIATIS, T., E.F. FRITSCH, and J. SAMBROCK (1982). Molecular cloning: a laboratory manual. Cold Spring Harbor Laboratory Press, New York.

McCLURE, M.A. (1990). In: New directions in biological control: Alternatives for suppressing agricultural pests and diseases. Alan R. Liss, Inc.

MOEHLE, C.M., R. TIZARD, S.K. LEMMON, J. SMART, and E.W. JONES (1987). Protease B of the lysosomelike vacuole of the yeast Saccharomyces cerevisiae is homologous to the subtilisin family of serine proteases. Mol. Cell. Biol. 7: 4390-4399.

MORRISSEY, J.H. (1981). Silver stainfor proteins in polyacrylamide gels: A modified procedure with enhanced uniform sensitvity. Analytical Biochemistry 117, 307-310.

NORTH, M.J. (1982). Comparative biochemistry of the proteinases of eucaryotic microorganism. Microbiological Review 46, 308-340.

RINDERKNECHT, H., GEOKAS, M.C., SILVERMAN, P. & HAVERBACK, B.J. (1968). A new ultrasensitive method for the determination of proteolytic activity. Clinica Acta 21, 197-203.

SAMSINAKOVA, A., MISIKOVA, S. & LEOPOLD, J. (1971). Action of enzymatic systems of Beauveria bassiana of the cuticle of greater Wax Moth larvae (Galleria mellonella). Journal of Invertebrate Pathology 18, 322-330.

SCHAAD, N.W. & WALKER, J.T. (1975). The use of densit-gradient centrifugation for the purification of eggs of Meloidogyne spp. Journal of Nemtology 7, 203-204.

SMITH, r.J., PEKRUL, S. & GRULA, E.A. (1981). Requirement for sequential enzymatic activities for penetrations of the integument of the corn earworm (Heliothis zea). Journal of Invertebrate Pathology 38, 335-344.

STEPANOV, V.M. & RUDENSKAYA, G.N. (1983). Proteinase affinity chromatography on Bacitracin-Sepharose. Journal of Applied Biochemistry 5, 420-428.

STIRLING, G.R. & MANKAU, R. (1979). Mode of parasitism of Meloidognye and other nematode eggs by Dactyllela oviparasitica. Journal of Nematolgy 11, 282-288.

ST. LEGER, R.J., COOPER, R.M. & CHARNLEY, A.K. (1986a). Cuticle-degrading enzymes of entomopathogenic fungi: Cuticle degradation in vitro by enzymes from entomopathogens. Journal of Invertebrate Pathology 47, 167-177.

ST. LEGER, R.J. CHARNLEY, A.K. & COOPER, R.M. (1986b). Culticle-degrading enzymes of entomopathogenic fungi: Mechanisms of interaction betwen pathogen enzymes and insect cuticle. Journal of Invertebrate Pathology 47, 295-302.

ST. LEGER, R.J., CHARNLEY, A.K. & COOPER, R.M. (1986c). Cuticle-degrading enzymes of entomopathogenic fungi: Synthesis in culture on cuticle. Journal of Invertebrate Pathology 48, 85-95.

ST. LEGER, R.J., COOPER, R.M. & CHARNLEY, A.K. (1987a). Production of cuticle-degradizing enzymes by the entomopathogen Metarhizium anisopliase during infection of cuticles from Calliphora vomitoria and Manduca sexta. Journal of General Microbiology 33, 1371-1382.

ST. LEGER, R.J., CHARNLEY, A.K. & COOPER, R.M. (1987b). Characterization of cuticle-degrading proteases produced by the entomopathogen Metarhizium anisopliae. Archives of Biochemistry and Biophysics 253, 1, 221-232.

ST. LEGER, R.J., DURRANDS, P.K., COOPER, R.M. & CHARNLEY, A.K. (1988). Regulation of production of proteolytic enzymes by the entomopathogenic fungus Metarhizium anisopliae. Archives of Microbiology 150, 413-416.

ST. LEGER, R.J., STAPLES, R.C. & ROBERTS, D.W. (1991). Changes in translatable mRNA species associated with nutrient deprivation and protease synthesis in Metarhizium anisopliae. Journal of General Microbiological 137, 807-815.

TATSUMI, H., Y. OGAWA, S. MURAKAWI, Y, ISHIDA, K. MURAKAMI, A. MASAKI, H. KAWABE, E. NAKANO, and H. MOTAI (1989). A full length cDNA clone for the alkaline protease from Aspergillus orizae: Structural analysis and expression in Saccharomyces cerevisiae. Mol. Gen. Genet. 219: 33-38.

VILLANUEVA, L.M. & Davide, R.G. (1984). Evaluation of several isolates of soil fungi for biological control of root-knot nematodes. Philippine Agriculture 67, 361-371.

ZAKI, F.A., and D.S. Bhatti (1990). Effect of castor (Ricinus communis) and Paecilomyces lilacinus on Meloidogyne javanica. Nematologica 36: 114-122.

## 7. Legends to figures

Figure 1: Protease activity of culture filtrates of Paecilomyces lilacinus ($10^8$ conidia) in 10 ml liquid medium (NM: minimal medium, CFM: corn four medium) to which different substrates were added: E: 2,500,000 Meloidogyne hapla eggs for MME and 760,000 M. hapla eggs for CFME, N: nitrogen ($NaNO_3$ (0.2 %) + asparagine (0.2 %)) and NG: nitrogen ($NaNO_3$ (0.2 %) + asparagine (0.2 %)) + glucose (2.0 %).

Figure 2: Protease activity / µg protein of culture filtrates of Paecilomyces lilacinus (mycelium after 6 days of growth in 250 ml MNNG) in 250 ml liquid medium (MN: minimal medium + nitrogen ($NaNO_3$ (0.2 %) + asparagine (0.2 %)) to which chitin was added as substrate. Glucose was added daily. CHIT : colloidal chitin (0.2 % or 1.0 % (w/v), GLUC : glucose (0.3 % (w/v) / day).

Figure 3: SDS-PAGE patterns of filtrates of 4 days old cultures of Paecilomyces lilacinus in minimal medium to which different substrates were added: Lane LG: Nitrogen + Glucose; Lane V: Vitellin; Lane C: Chitin; Lane E: Eggs; Lane M: Markers, Molecular weight of markers are indicated.

Figure 4a: Protease activity of fractions of Bactricine-Sepharose column to which concentrated culture filtrate of Paecilomyces lilacinus, grown in minimal medium with chitin as substrate, was added. Elution was started at fraction 7. Procedure was described under Materials & Methods.

Figure 4b: SDS-PAGE patterns of fractions of Bacitracine-Sepharose column to which concentrated culture filtrate of Paecilomyces lilacinus grown in minimal medium with chitin as substrate, was added. Procedure was described under Materials & Methods.

Lane CF: total culture filtrate with chitin as substrate

Lane 1-11: fraction 1-11.

Lane M: Markers. Molecular weight of markers are indicated.

Figure 5: Protease activity of purified protease (1.875 µg) of Paecilomyces lilacinus at different pH. Measurements were performed in triplicate. Protease was purified as described under Materials & Methods from culture filtrate of P. lilacinus in minimal medium with vitellin as substrate.

Figure 6: Protease activity of purified protease (1.875 µg) of Paecilomyces lilacinus at different temperatures. Measurements were performed in triplicate. Protease ws purified as described under Materials & Methods from culture filtrate of P. lilacinus in minimal medium with vitellin as substrate.

Figure 7: Protease activity or purified protease (0.47 µg) of Paecilomyces lilacinus after incubation for 40 minutes at 4 °C with different inhibitors, SH-reducing agents and metal ions. Measurements were performed in triplicate. Final inhibitor concentration was 1 mM. Protease was purified as described under Materials & Methods from culture filtrate of P. lilacinus in minimal medium with vitellin as substrate. Protease-inhibitors were respectively none, PMSF, 1,10-phenanthroline, Pepstatine, E64, DTT, Cysteine, $CaCl_2$, $MgCl_2$ and EDTA.

Legends to Tables

Table 1

Protease activity and protein content of filtrates of 4 days old culture of Paecilomyces lilacinus ($2x10^8$ conidia) in 200 ml liquid medium (NM: minimal medium) to which different substrates were added:

C : colloidal chitin (0.2 % (w/v)),

E : 2.250.000 Meloidogyne hapla eggs,

V : vitellin (0.2 % (w/v)),

NG: nitrogen ($NaNO_3$ (0.2 % (w/v)) + asparagine (0.2 %) (w/v) + glucose (2.0 % (w/v).

Culture filtrates were concentrated to 10 ml and dialyzed against demineralized water.

Table 2

Protease activity and glucose-concentration of culture filtrate, and weighth of mycelium of 4 days old cultures of Paecilomyces lilacinus ($1.5x10^8$ conidia) in 10 ml liquid medium (MM: minimal medium) to which different substrates were added:

C : colloidal chitin (0.2 % (w/v)),

E : 450,000 Meloidogyne hapla, eggs,

N : nitrogen ($NaNO_3$ (0.2 % (w/v)) + asparagine (0.2 % (w/v)),

G : glucose (2.0 % (w/v)).

Culture filtrate was supplemented to 15 ml with sterile demineralized water.

Sequenceprotokoll No. 1

Type: Nucleotide

Sequence lenght: 1316 base pairs

Strandedness: double

Topology: linear

Molecular Type: genomic DNA

Original source Organism: Paecilomyces lilacinus

Seq ID NO.1

```
    GGCACGAGCTCCTCTCCTGACGCCCCGCGCGCCAGCAGCTCATCAACGGCAAGTACATTG
  1 ---------+---------+---------+---------+---------+---------+ 60
    CCGTCCTCGAGGAGAGGACTGCGGGGCGCGCGGTCGTCGAGTAGTTGCCGTTCATGTAAC

    TCAAGTTCAAGGACGGCATGTCCATCGCCTCTGTCGACAAGACTGTCAGCGCTCTGTCCT
 61 ---------+---------+---------+---------+---------+---------+ 120
    AGTTCAAGTTCCTGCCGTACAGGTAGCGGAGACAGCTGTTCTGACAGTCGCGAGACAGGA

    CCAACGCCGACCGCGTCTACAACCACATTTTCCGAGGCTTCGCCGCAACCTGCAATGCCA
121 ---------+---------+---------+---------+---------+---------+ 180
    CCTTCCGGCTGGCGCAGATGTTGGTGTAAAAGGCTCCGAAGCGGCGTTGGACGTTACGGT

    ACGACCTCAAGACCCTGCGCGACCACCCTGATGTCGAGTACATTGAGCAGGATGCCATAA
181 ---------+---------+---------+---------+---------+---------+ 240
    TCCTCCACTTCTGGGACGCGCTGGTGGGACTACACCTCATGTAACTCGTCCTACGGTATT

    TCACCATCAACGCGTACACTCAGCAGCCCGGCGCCCCCTGGGGTCTCGGACGCATCTCTC
241 ---------+---------+---------+---------+---------+---------+ 300
    AGTGGTAGTTGCGCATGTGAGTCGTCGGGCCGCGGGGGACCCCAGAGCCTGCGTAGAGAG

    ACCGCAGCAAGGGTAGCACCACATACGAGTATGATACCAGCGGCGGCAGTGGCACCTGCG
301 ---------+---------+---------+---------+---------+---------+ 360
    TGGCGTCGTTCCCATCGTCCTGTATGCTCATACTATGGTCGCCGCCGTCACCGTGGACGC

    CCTATGTCATCGACACTGGCGTCGAGGCTTCGCACCCCGAGTTCGAGGGCCGCGCCAGCC
361 ---------+---------+---------+---------+---------+---------+ 420
    GGATACAGTAGCTGTGACCGCAGCTCCGAAGCGTGGGGCTCAAGCTCCCGGCGCGGTCGG

    ACCAGATCAAGAGCTTCATCAGCGGCCAGAACACCGACGGCAACGGCCATGGCACTCACT
421 ---------+---------+---------+---------+---------+---------+ 480
    TGGTCTAGTTCTCGAAGTAGTCGCCGGTCTTGTGGCTGCCGTTGCCGGTACCGTGAGTGA

    GCGCCGGCACCATCGGCTCCAAGACGTACGGTGTTGCCAAGAAGACCAAGATCTACGGTG
481 ---------+---------+---------+---------+---------+---------+ 540
    CGCGGCCGTGGTAGCCGAGGTTCTGCATGCCACAACGGTTCTTCTGGTTCTAGATGCCAC
```

```
     TCAAGGTCCTCGACAACTCGGGCTCCGCTCATACTCGGGCATCATTTCTCCGGTATGGAC
 541 ---------+---------+---------+---------+---------+---------+ 600
     AGTTCCAGGAGCTGTTGAGCCCGAGGCGAGTATGAGCCCGTAGTAAAGAGGCCATACCTG

     TTTCCCGTTCAGGACTCCAAGATCGCGCAGCTGCCCCAAGGGTGTCGTCGCCAATATGTC
 601 ---------+---------+---------+---------+---------+---------+ 660
     AAACGGCAAGTCCTGAGGTTCTAGCGCGTCGACGGGGTTCCCACAGCAGCGGTTATACAG

     TCTGGCCGCTGCAAAGGCTCAGTCTCAACGACGGTGCCGCTGCCATGATCAGGCGCGCGT
 661 ---------+---------+---------+---------+---------+---------+ 720
     ACACCGGCGACGTTTCCGAGTCAGAGTTGCTGCCACGGCGACGGTACTAGTCCGCGCGCA

     CTTCCTCCCCCTCCCGCTGGCAACGACAACGTAACGCCGCCAACTACTCCCCTGCCTCTG
 721 ---------+---------+---------+---------+---------+---------+ 780
     GAAGGAGGGGGAGGGCGACCGTTGCTGTTGCATTGCGGCGGTTGATGAGGGGACGGAGAC

     AGCCGACTGTTTGCACCCTCGGCGCCACCACCTCTTCTGATCCGCGATCTTCGTTCTCCA
 781 ---------+---------+---------+---------+---------+---------+ 840
     TCGGCTGACAAACGTGGGAGCCGCGGTGGTGGAGAAGACTAGGCGCTAGAAGCAAGAGGT

     ACTACGGCAATCTCCTCGACATCTTCGCCCCGGGTAGCAACATTCTGTCCACCTGGTTCG
 841 ---------+---------+---------+---------+---------+---------+ 900
     TGATGCCGTTAGAGGAGCTGTAGAAGCGGGGCCCATCGTTGTAAGACAGGTGGACCAAGC

     GTGGCACTACCAACACCATCTCTGGTACTTCCATGGCCACTCCCCACATTGTTGGTCTCG
 901 ---------+---------+---------+---------+---------+---------+ 960
     CACCGTGATGGTTGTGGTAGAGACCATGAAGGTACCGGTGAGGGGTGTAACAACCAGAGC

     GCGCCTACCTCGCCGGTCTGGAGGGTTTCCCCGGCGCCCAGGCGCTCTGCAAGCGCATCC
 961 ---------+---------+---------+---------+---------+---------+ 1020
     CGCGGATGGAGCGGCCAGACCTCCCAAAGGGGCCGCGGGTCCGCGAGACGTTCGCGTAGG

     AGACCCTTTCTTACTAAGAACGTCCTCACCGCATTCCCAGCGGCACTTGTCAACTACCTT
1021 ---------+---------+---------+---------+---------+---------+ 1080
     TCTGGGAAAGAATGATTCTTGCAGGAGTGGCGTAAGGGTCGCCGTGAACAGTTGATGGAA

     CGCCTTCAACGGCAAACCCCAGCGGCTAAATGCTACAAGAATGAGGACTCCTCGACGACC
1081 ---------+---------+---------+---------+---------+---------+ 1140
     GCGGAAGTTGCCGTTTGGGGTCGCCGATTTACGATGTTCTTACTCCTGAGGAGCTGCTGG

     TTTATCCCAGCCATGACCTTTTTTGGAGCATATCAACAGCGGCGAGATCCTCTTGGGACA
1141 ---------+---------+---------+---------+---------+---------+ 1200
     AAATAGGGTCGGTACTGGAAAAAACCTCGTATAGTTGTCGCCGCTCTAGGAGAACCCTGT

     AACCTAAGTATGTGGCATTGAACTGCGCTCCTGTCATATACTCCGCGCAAGGAAGAGTAA
1201 ---------+---------+---------+---------+---------+---------+ 1260
     TTGGATTCATACACCGTAACTTGACGCGAGGACAGTATATGAGGCGCGTTCCTTCTCATT

     AAAGTGGACATGTCTTTTAAATAGCACCAATACATATCTTATGAGGCCTCGAAAAAAAAA
1261 ---------+---------+---------+---------+---------+---------+ 1320
     TTTCACCTGTACAGAAAATTTATCGTGGTTATGTATAGAATACTCCGGAGCTTTTTTTTT

     AAAAAAAAAAAAAAAA
1321 ---------+------ 1336
     TTTTTTTTTTTTTTTT
```

TABLE 1

| Medium | Protease Activity ($A_{595}$ $ml^{-1}$ $hr^{-1}$) | Protein content ($\mu g$ $ml^{-1}$) | Specific Protease Activity ($A_{595}$ $\mu g^{-1}$ $hr^{-1}$) |
|---|---|---|---|
| MMNG | 4.24 | 47.5 | 0.089 |
| MMC | 23.85 | 104.2 | 0.229 |
| MME | 21.83 | 35.3 | 0.618 |
| MMV | 93.79 | 303.9 | 0.309 |

TABLE 2

| Medium | Protease Activity ($A_{595}$ $ml^{-1}$ $hr^{-1}$) | Glucose Concentration (%) | Weight Mycelium (mg) | Specific Protease Activity ($A_{595}$ $ml^{-1}$ $hr^{-1}$ mg $mycelium^{-1}$) x 1000 |
|---|---|---|---|---|
| MMG | 0.06 | 2.12 | 20 | 2.79 |
| MMGE | 5.79 | 1.80 | 50 | 115.76 |
| MMGC | 5.64 | 1.04 | 100 | 56.36 |
| MMNG | 1.37 | 0.29 | 180 | 7.59 |
| MMNGE | 5.38 | 0.006 | 200 | 26.91 |
| MMNGC | 1.46 | 0.002 | 350 | 4.17 |

SEQUENCE LISTING

(1) GENERAL INFORMATION:

(i) APPLICANT:
(A) NAME: Research Institut for Plant Protection (IPO-DLO)
(B) STREET: Binnenhaven 12 / Postbus 9060
(C) CITY: Wageningen
(E) COUNTRY: Netherland
(F) POSTAL CODE (ZIP): 6700 GW
(G) TELEPHONE: 08370-76000
(H) TELEFAX: 08370-10113
(I) TELEX: 45888 intas nl

(ii) TITLE OF INVENTION: New alkaline serine protease of Paecilomyces lilacinus

(iii) NUMBER OF SEQUENCES: 13

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 1336 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(A) ORGANISM: Paecilomyces lilacinus

(xi) SEQUENCE DESCRIPTION: SEQ ID NO: 1:

GGCACGAGCT CCTCTCCTGA CGCCCCGCGC GCCAGCAGCT CATCAACGGC AAGTACATTG 60

TCAAGTTCAA GGACGGCATG TCCATCGCCT CTGTCGACAA GACTGTCAGC GCTCTGTCCT 120

CGAAGGCCGA CCGCGTCTAC AACCACATTT TCCGAGGCTT CGCCGCAACC TGCAATGCCA 180

ACGACCTCAA GACCCTGCGC GACCACCCTG ATGTCGAGTA CATTGAGCAG GATGCCATAA 240

TCACCATCAA CGCGTACACT CAGCAGCCCG GCGCCCCCTG GGGTCTCGGA CGCATCTCTC 300

ACCGCAGCAA GGGTAGCACC ACATACGAGT ATGATACCAG CGGCGGCAGT GGCACCTGCG 360

CCTATGTCAT CGACACTGGC GTCGAGGCTT CGCACCCCGA GTTCGAGGGC CGCGCCAGCC 420

ACCAGATCAA GAGCTTCATC AGCGGCCAGA ACACCGACGG CAACGGCCAT GGCACTCACT 480

GCGCCGGCAC CATCGGCTCC AAGACGTACG GTGTTGCCAA GAAGACCAAG ATCTACGGTG 540

TCAAGGTCCT CGACAACTCG GGCTCCGCTC ATACTCGGGC ATCATTTCTC CGGTATGGAC 600

```
TTTGCCGTTC AGGACTCCAA GATCGCGCAG CTGCCCCAAG GGTGTCGTCG CCAATATGTC    660
TCTGGGCGGT GGAAAGGCTC AGTCTCAACG ACGGTGCCGC TGCCATGATC AGGCGCGCGT    720
CTTCCTCGCC GTCGCGCTGG CAACGACAAC GTAACGCCGC CAACTACTCC CCTGCCTCTG    780
AGCCGACTGT TTGCACCGTC GGCGCCACCA CCTCTTCTGA TCCGCGATCT TCGTTCTCCA    840
ACTACGGCAA TCTCGTCGAC ATCTTCGCCC CGGGTAGCAA CATTCTGTCC ACCTGGTTCG    900
GTGGCACTAC CAACACCATC TCTGGTACTT CCATGGCCAC TCCCCACATT GTTGGTCTCG    960
GCGCCTACCT CGCCGGTCTG GAGGGTTTCC CCGGCGCCCA GGCGCTCTGC AAGCGCATCC   1020
AGACCCTTTC TTACTAAGAA CGTCCTCACC GCATTCCCAG CGGCACTTGT CAACTACCTT   1080
CGCCTTCAAC GGCAAACCCC AGCGGCTAAA TGCTACAAGA ATGAGGACTC CTCGACGACC   1140
TTTATGCCAG CCATGACCTT TTTTGGAGCA TATCAACAGC GGCGAGATCC TCTTGGGACA   1200
AAGGTAAGTA TGTGGCATTG AACTGCGCTC CTGTCATATA CTCCGCGCAA GGAAGAGTAA   1260
AAAGTGGACA TGTCTTTTAA ATAGCACCAA TACATATCTT ATGAGGCCTC GAAAAAAAAA   1320
AAAAAAAAAA AAAAAA                                                   1336
```

(2) INFORMATION FOR SEQ ID NO: 2:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: protein

(iii) HYPOTHETICAL: NO

(v) FRAGMENT TYPE: N-terminal

(vi) ORIGINAL SOURCE:
(A) ORGANISM: Aspergillus oryzae

(ix) FEATURE:
(A) NAME/KEY: Protein
(B) LOCATION: 1..15

(xi) SEQUENCE DESCRIPTION: SEQ ID NO: 2:

```
Gly Leu Thr Thr Gln Lys Ser Ala Pro Trp Gly Leu Ser Ile Ser
1               5                   10                  15
```

(2) INFORMATION FOR SEQ ID NO: 3:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: protein

(iii) HYPOTHETICAL: NO

(v) FRAGMENT TYPE: N-terminal

(vi) ORIGINAL SOURCE:
(A) ORGANISM: Saccharomyces cerevisiae

(A) LENGTH: 16 amino acids
(B) TYPE: amino acid
(C) STRANDEDNESS: single
(D) TOPOLOGY: linear

(ii) MOLECULE TYPE: protein

(iii) HYPOTHETICAL: NO

(v) FRAGMENT TYPE: N-terminal

(vi) ORIGINAL SOURCE:
(A) ORGANISM: Paecilomyces lilacinus

(ix) FEATURE:
(A) NAME/KEY: Protein
(B) LOCATION: 1..16

(xi) SEQUENCE DESCRIPTION: SEQ ID NO: 6:

```
Ala Tyr Thr Gln Gln Pro Gly Ala Pro Trp Gly Leu Gly Arg Ile Ser
1               5                   10                  15
```

(2) INFORMATION FOR SEQ ID NO: 7:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: protein

(iii) HYPOTHETICAL: NO

(v) FRAGMENT TYPE: internal

(vi) ORIGINAL SOURCE:
(A) ORGANISM: Aspergillus oryzae

(ix) FEATURE:
(A) NAME/KEY: Protein
(B) LOCATION: 1..12

(xi) SEQUENCE DESCRIPTION: SEQ ID NO: 7:

```
Asp Ser Ile Gly His Gly Thr His Val Ser Gly Thr
1               5                   10
```

(2) INFORMATION FOR SEQ ID NO: 8:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: protein

(iii) HYPOTHETICAL: NO

(iii) ANTI-SENSE: NO

(v) FRAGMENT TYPE: internal

(vi) ORIGINAL SOURCE:

(A) ORGANISM: Saccharomyces cerevisiae

(ix) FEATURE:
(A) NAME/KEY: Protein
(B) LOCATION: 1..12

(xi) SEQUENCE DESCRIPTION: SEQ ID NO: 8:

```
Asp Gly Asn Gly His Gly Thr His Cys Ala Gly Thr
1               5                   10
```

(2) INFORMATION FOR SEQ ID NO: 9:

(i) SEQUENCE CHARACTERISTICS:
(A) LENGTH: 11 amino acids
(B) TYPE: amino acid
(C) STRANDEDNESS: single
(D) TOPOLOGY: linear

(ii) MOLECULE TYPE: protein

(iii) HYPOTHETICAL: NO

(v) FRAGMENT TYPE: internal

(vi) ORIGINAL SOURCE:
(A) ORGANISM: Yarrowia lipolytica

(ix) FEATURE:
(A) NAME/KEY: Protein
(B) LOCATION: 1..11

(xi) SEQUENCE DESCRIPTION: SEQ ID NO: 9:

```
Asp Leu Leu Gly His Thr His Val Ala Gly Thr
1               5                   10
```

(2) INFORMATION FOR SEQ ID NO: 10:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: protein

(iii) HYPOTHETICAL: NO

(v) FRAGMENT TYPE: internal

(vi) ORIGINAL SOURCE:
(A) ORGANISM: Tritirachium album

(ix) FEATURE:
(A) NAME/KEY: Protein
(B) LOCATION: 1..12

(xi) SEQUENCE DESCRIPTION: SEQ ID NO: 10:

```
Asp Gly Asn Gly His Gly Thr His Cys Ala Gly Thr
1               5                   10
```

(2) INFORMATION FOR SEQ ID NO: 11:

(i) SEQUENCE CHARACTERISTICS:

```
        (A) LENGTH: 12 amino acids
        (B) TYPE: amino acid
        (C) STRANDEDNESS: single
        (D) TOPOLOGY: linear

   (ii) MOLECULE TYPE: protein

    (v) FRAGMENT TYPE: internal

   (vi) ORIGINAL SOURCE:
        (A) ORGANISM: Paecilomyces lilacinus

   (ix) FEATURE:
        (A) NAME/KEY: Protein
        (B) LOCATION: 1..12

   (xi) SEQUENCE DESCRIPTION: SEQ ID NO: 11:

    Asp Gly Asn Gly His Gly Thr His Cys Ala Gly Thr
    1               5                   10

(2) INFORMATION FOR SEQ ID NO: 12:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

   (ii) MOLECULE TYPE: DNA (genomic)

   (vi) ORIGINAL SOURCE:
        (A) ORGANISM: Paecilomyces lilacinus

   (ix) FEATURE:
        (A) NAME/KEY: 3'clip
        (B) LOCATION: 1..26

   (xi) SEQUENCE DESCRIPTION: SEQ ID NO: 12:

GCNTAYACNC ARCARCCNGG NGCNCC                                        26

(2) INFORMATION FOR SEQ ID NO: 13:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear

   (ii) MOLECULE TYPE: DNA (genomic)

   (vi) ORIGINAL SOURCE:
        (A) ORGANISM: Paecilomyces lilacinus

   (ix) FEATURE:
        (A) NAME/KEY: -
        (B) LOCATION: 1..26

   (xi) SEQUENCE DESCRIPTION: SEQ ID NO: 13:

GTNCCNGCRC ARRGNGTNCC RTGNCCRTTN CC                                 32
```

## Claims

**1.** A protease from Paecilomyces lilacinus having
   - a molecular weight of 20,00 to 200,000 Dalton

- an isoelectric point at a pH between 8 and 12,
- a pH optimum in a range from 9 and 12 and
- a substrate specificity towards surface structures of plant parasitic nematodes.

**2.** A protease according to claim 1 having a molecular weight of 25,000 to 100,000 Dalton.

**3.** A protease acoording to claim 1 having an isoelectric point which is at a pH of 9 to 11.

**4.** A protease according to claim 1 having an temperature optimum between 40 and 80 °C, preferably between 50 and 70 °C, especially between 55 and 65 °C.

**5.** A process for the preparation of the protease according to claim 1, which comprises cultivation of Paecilomyces lilacinus and isolation of said protease.

**6.** The use of the protease according to claim 1 for the controll of plant parasitic nematodes.

**7.** A gene for a protease having the DNA sequence shown in sequence protocoll 1.

**8.** A plasmid containing a gene as claimed in claim 7.

**9.** A microorganism containg a gene as claimed in claim 7.

**10.** A plant or a plant cell containing a gene as claimed in claim 7.

Figure 1

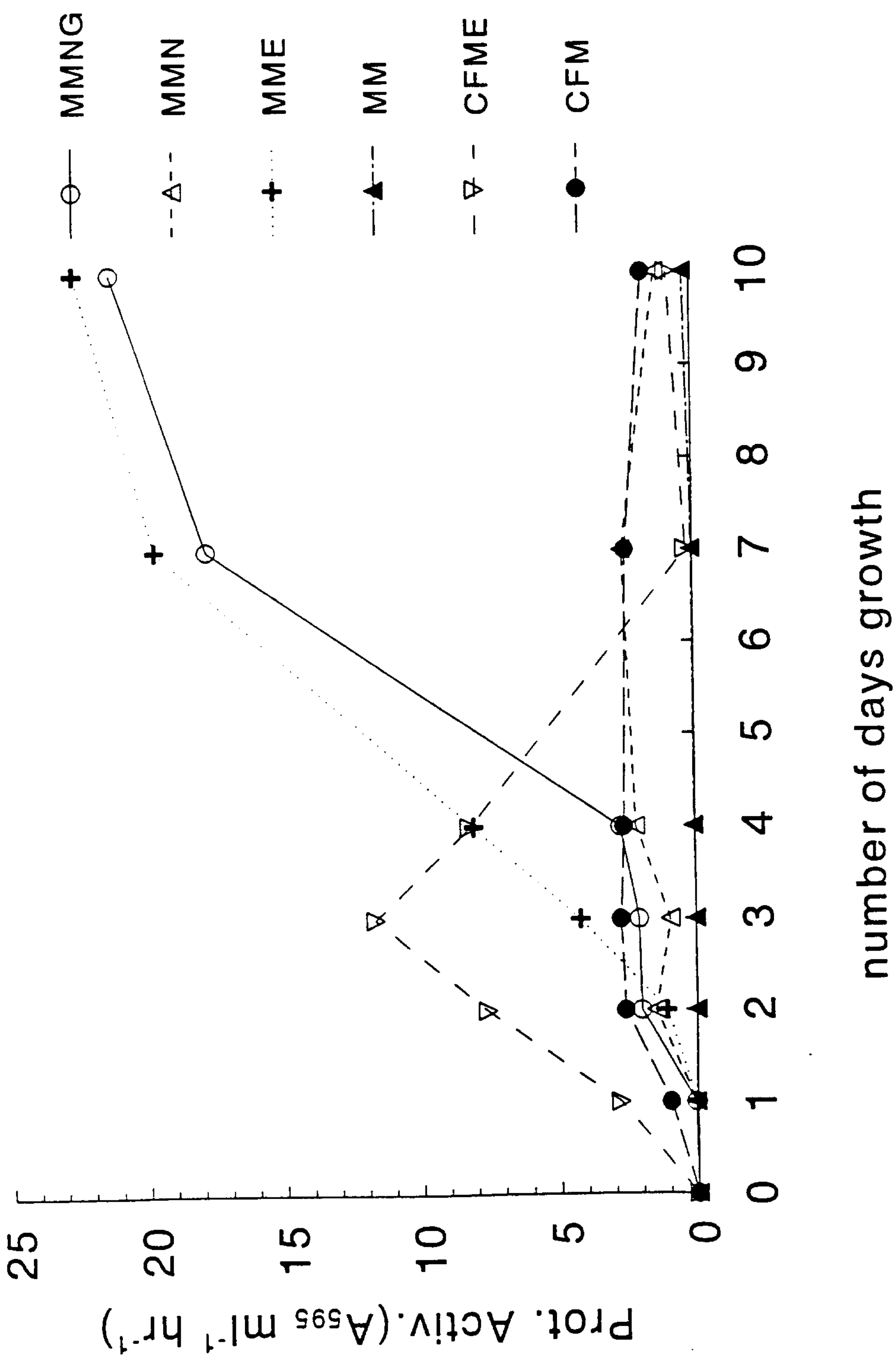
Prot. Activ.($A_{595}$ $ml^{-1}$ $hr^{-1}$)
0
5
10
15
20
25
0
1
2
3
4
5
6
7
8
9
10
number of days growth
MMNG
MMN
MME
MM
CFME
CFM

Figure 2

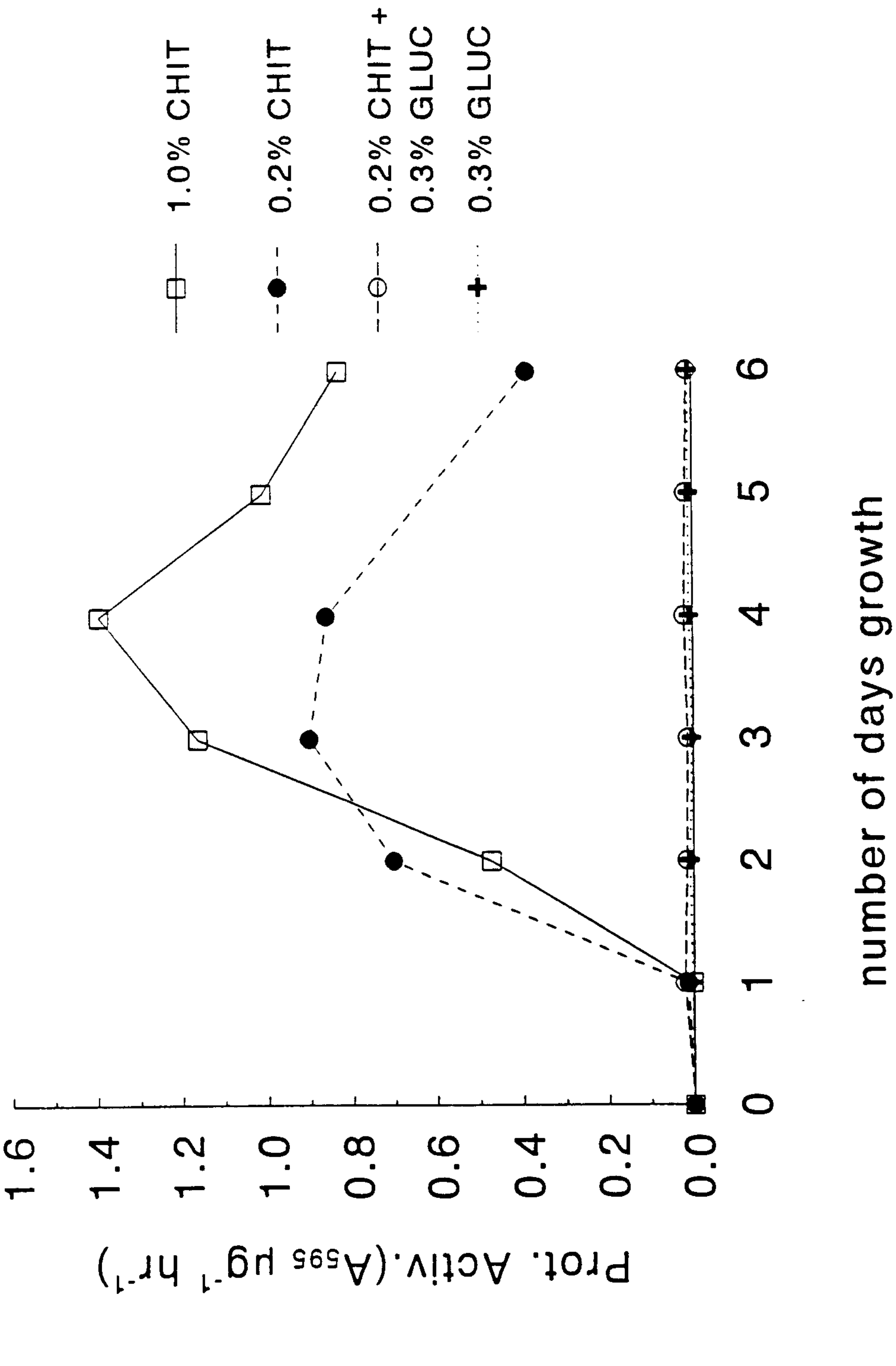
Prot. Activ.($A_{595}$ µg$^{-1}$ hr$^{-1}$)
0.0
0.2
0.4
0.6
0.8
1.0
1.2
1.4
1.6
0
1
2
3
4
5
6
number of days growth
1.0% CHIT
0.2% CHIT
0.2% CHIT + 0.3% GLUC
0.3% GLUC

Figure 3

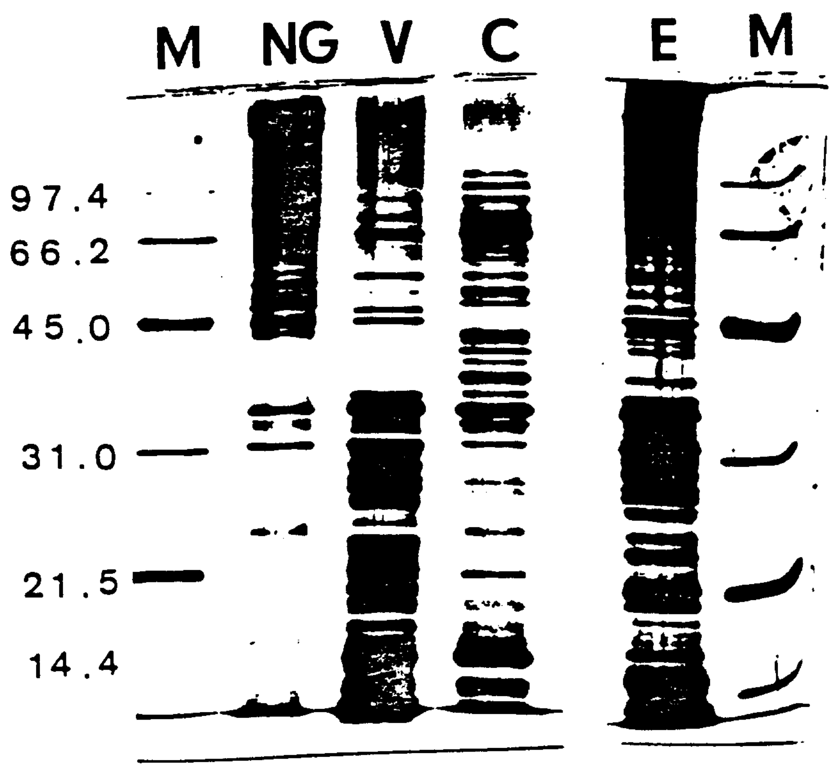

Figure 4a

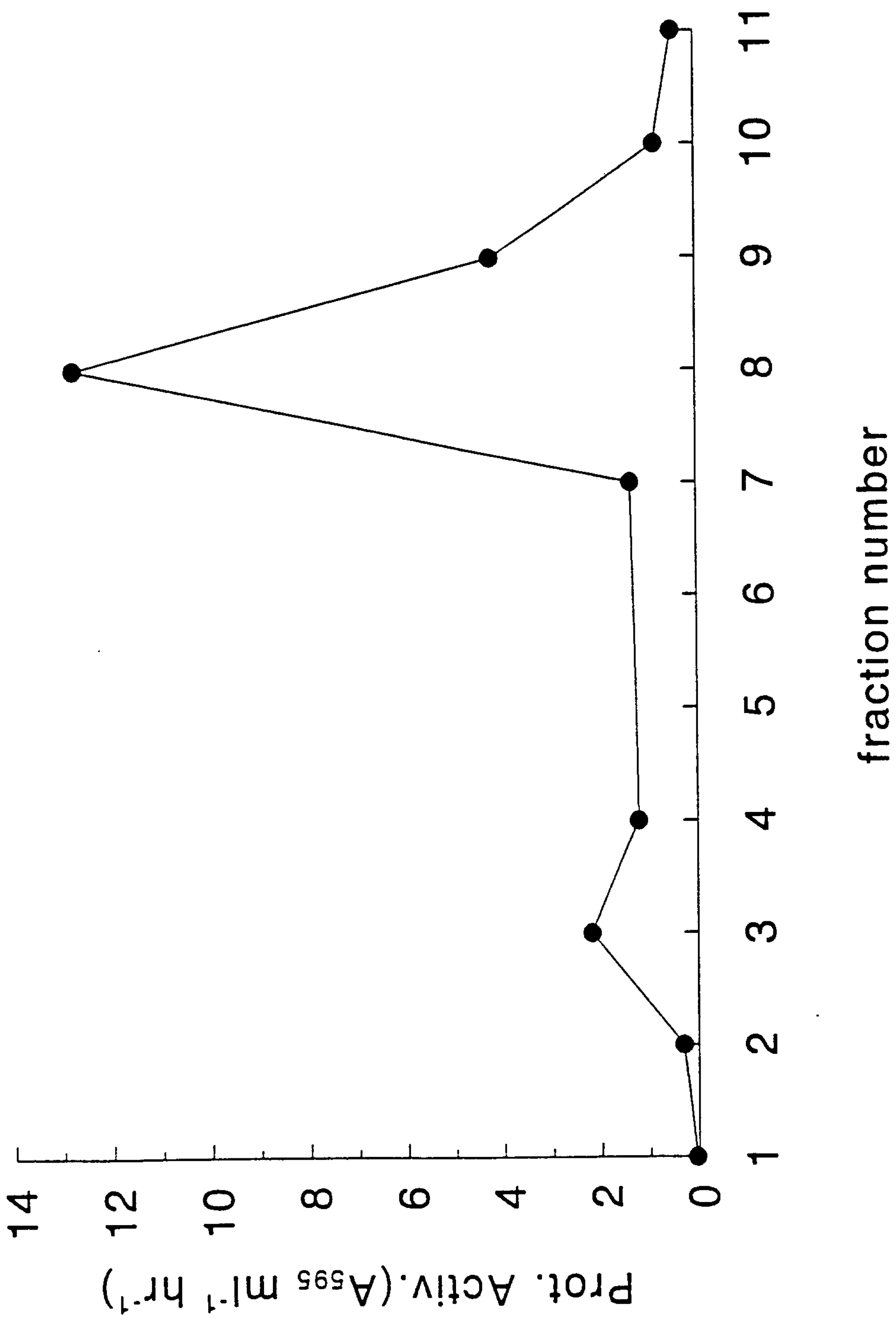
Prot. Activ.($A_{595}$ $ml^{-1}$ $hr^{-1}$)
0
2
4
6
8
10
12
14
1
2
3
4
5
6
7
8
9
10
11
fraction number

Figure 4b

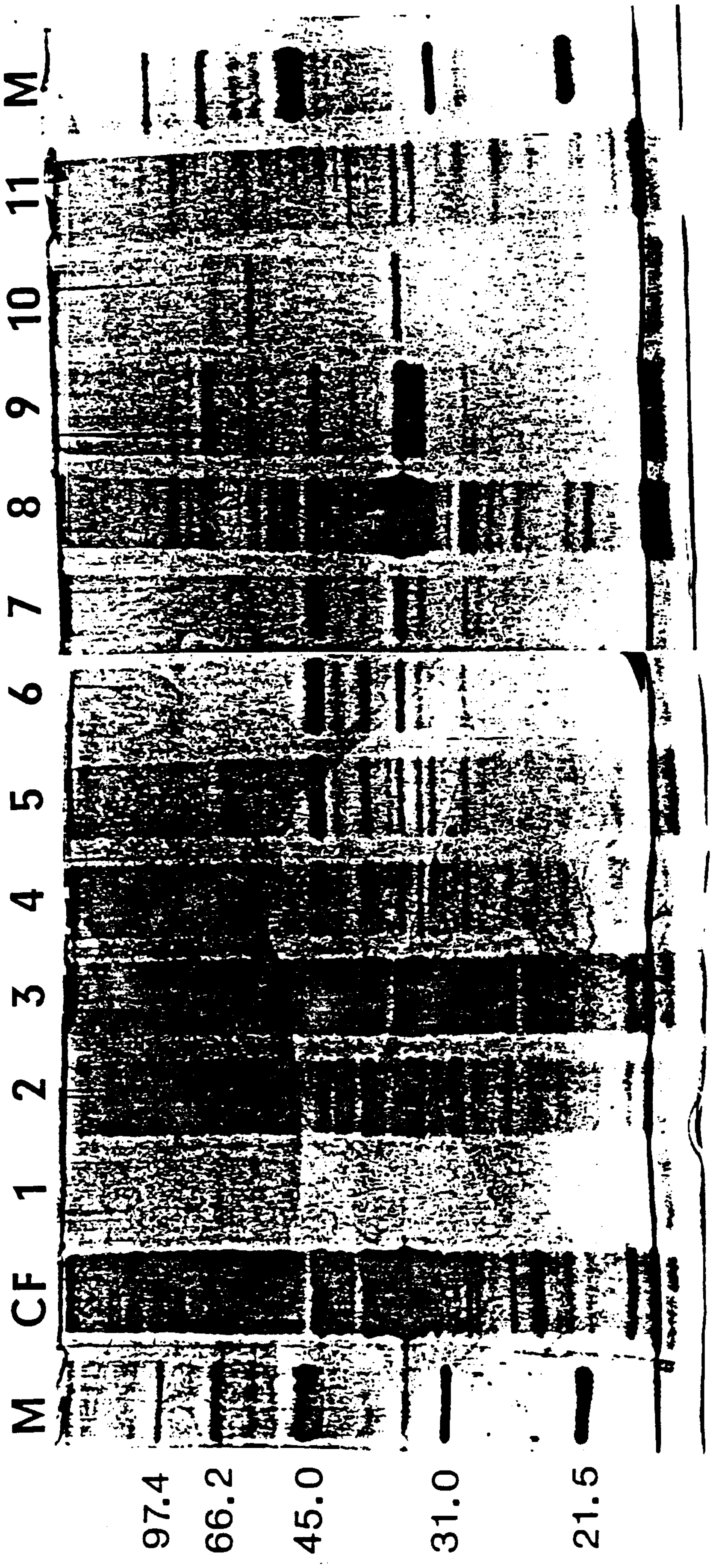

Figure 5

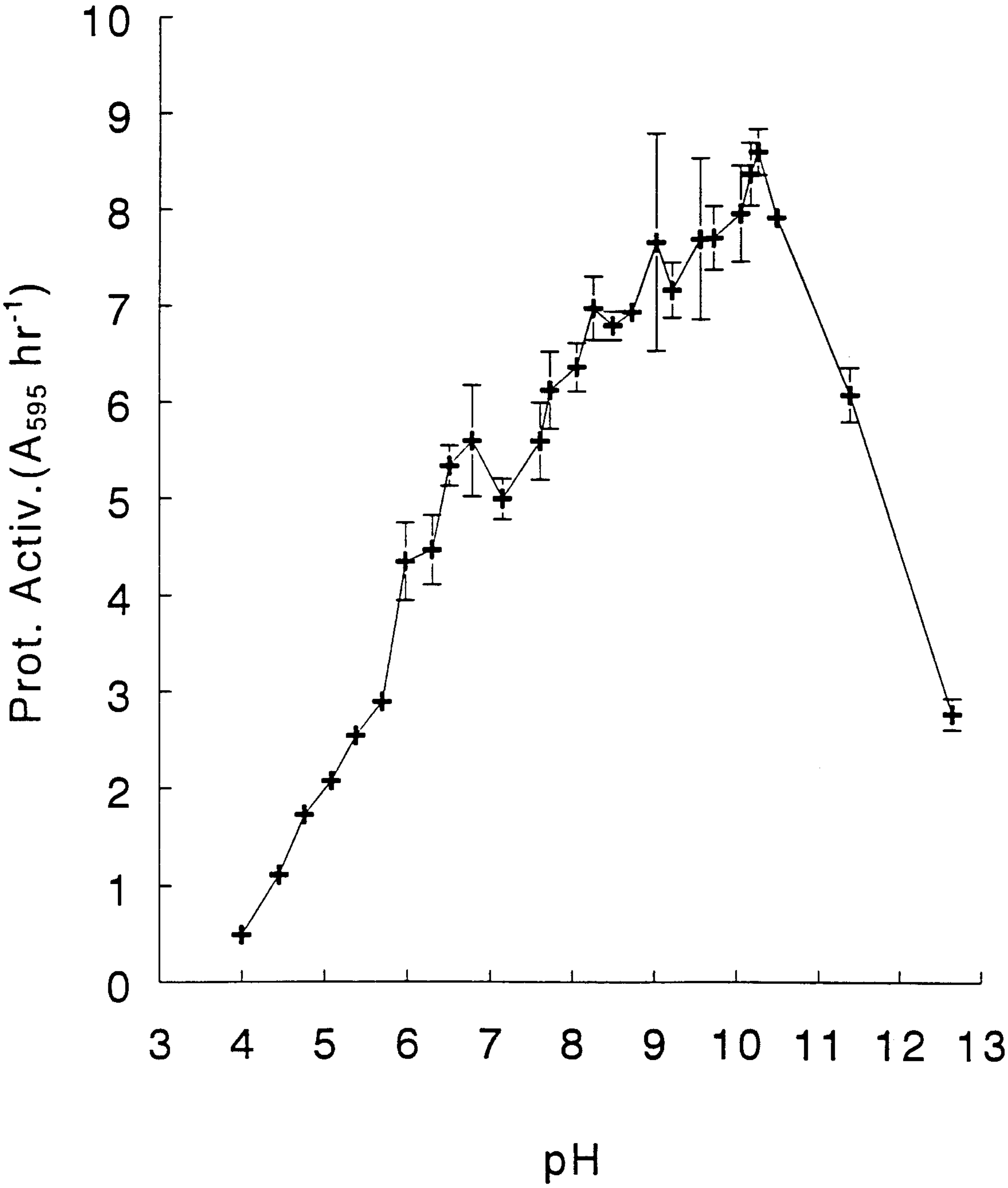

Figure 6

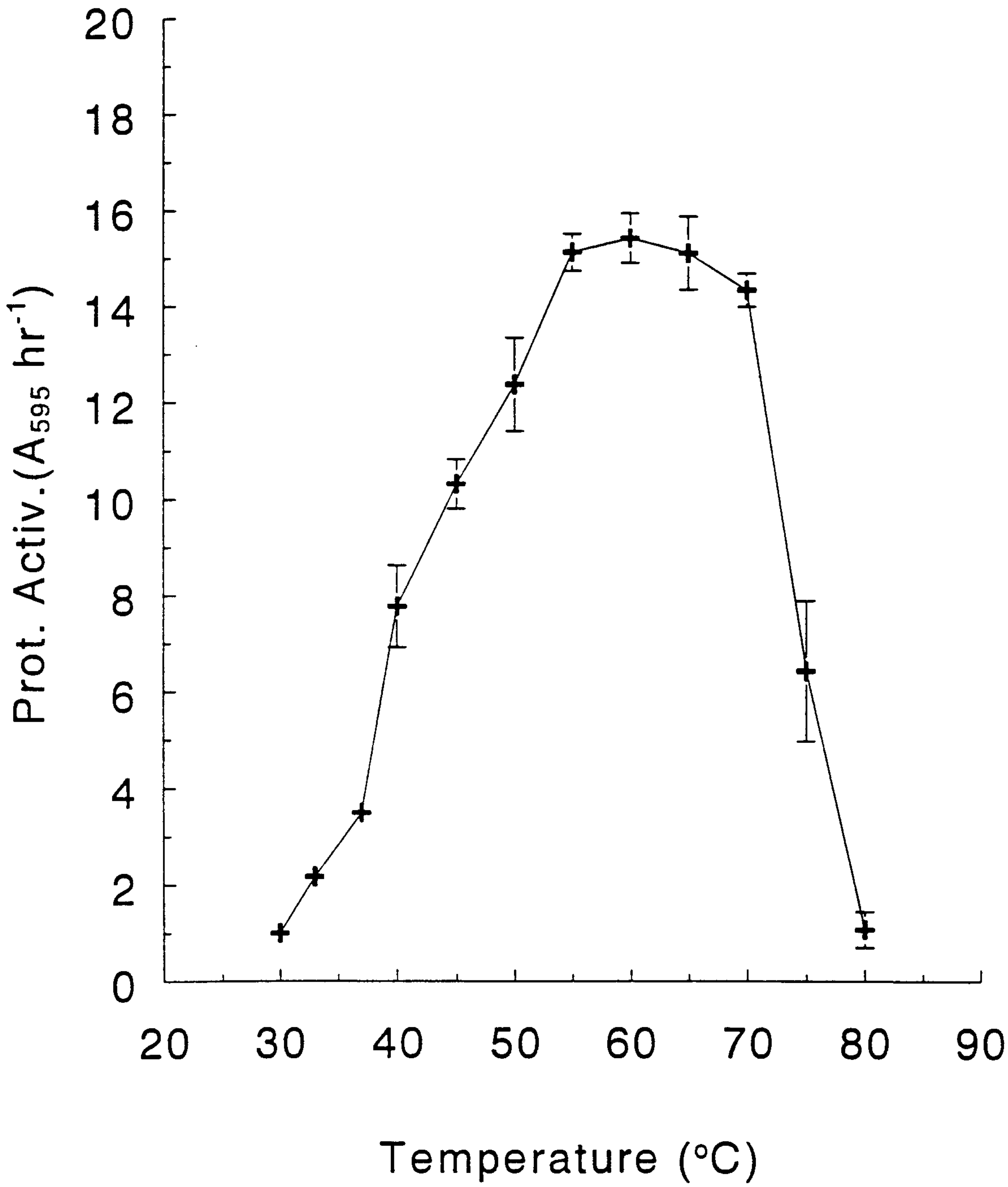
Prot. Activ.(A595 hr-1)
0
2
4
6
8
10
12
14
16
18
20
20
30
40
50
60
70
80
90
Temperature (°C)

Figure 7

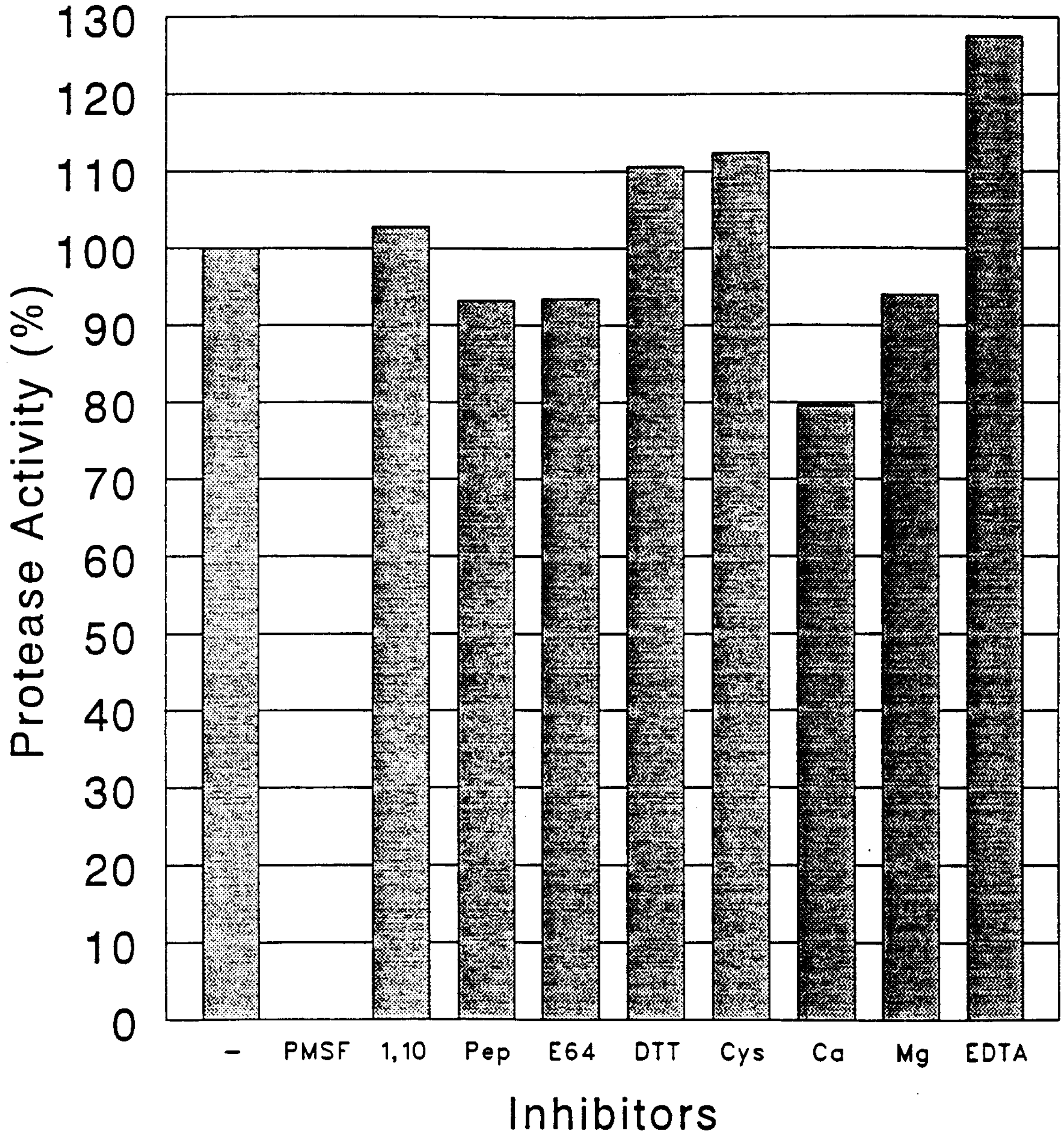
130
120
110
100
90
80
70
60
50
40
30
20
10
0
Protease Activity (%)
-
PMSF
1,10
Pep
E64
DTT
Cys
Ca
Mg
EDTA
Inhibitors

```
                                         (Cys)
                                         (His)
Ala-Tyr-Thr-Gln-Gln-Pro-Gly-Ala-Pro-Trp-Gly-Leu-Gly-Arg-Ile-Ser

                                    CAY             AGR AGY
GCN-TAY-ACN-CAR-CAR-CCN-GGN-GCN-CCN-TGB-GGN-CTN-GGN-CGN-ATH-TCN
**  **  **  **  **  **  **  **  **
GCI-TAY-ACI-CAR-CAR-CCI-GGI-GCI-CC   (Primer A)


                 *
-------Gly-Asn-Gly-His-Gly-Thr-His-Cys-Ala-Gly-Thr--------

5' -  GGN-AAY-GGN-CAY-GGN-ACN-CAY-TGY-GCN-GGN-ACN  - 3'
    **  **  **  **  **  **  **  **  **  **  **
3' -  CCI-TTR-CCI-GTR-CCI-TGI-GTR-ACR-CGI-CCI-TG   - 5' (Primer B)
```

Figure 8

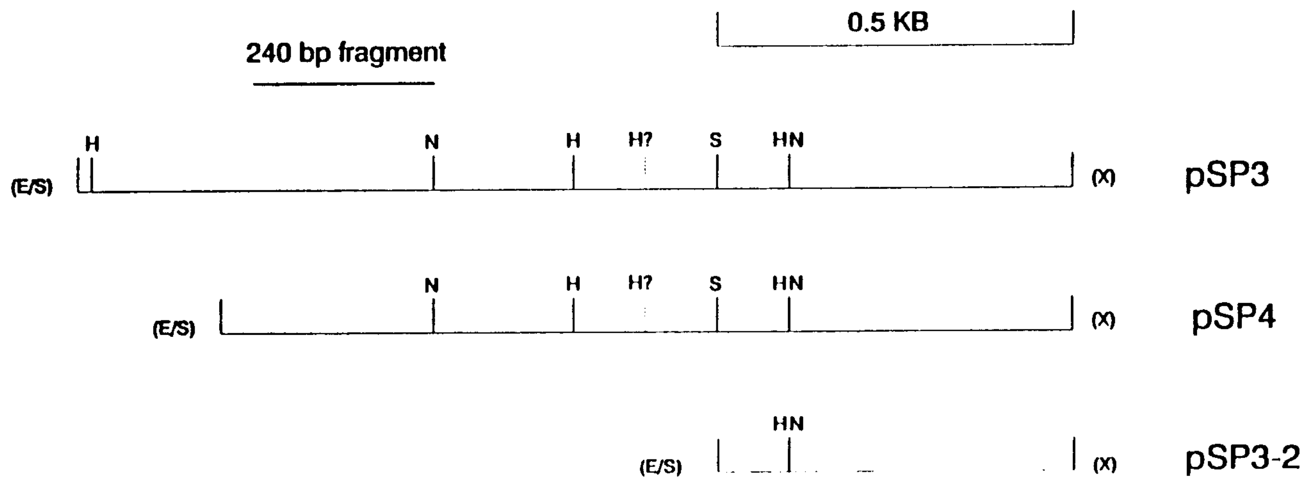


**Figure 4**. Restriction map of pSP3, pSP4, pSP3-2. The probable location of the 240-bp PCR-generated fragment is shown. E, EcoRI; H, HincII; H?, HincII, location uncertain; N, NcoI; S, SmaI; X, XhoI. No AvaII, BamHI, NotI, NruI, PstI, SacI, SacII sites, no internal EcoRI and XhoI sites.

Figure 9

```
      GGCACGAGCTCCTCTCCTGACGCCCCGCGCGCCAGCAGCTCATCAACGGCAAGTACATTG
   1 ---------+---------+---------+---------+---------+---------+ 60
      CCGTGCTCGAGGAGAGGACTGCGGGGCGCGCGGTCGTCGAGTAGTTGCCGTTCATGTAAC

      TCAAGTTCAAGGACCGCATGTCCATCGCCTCTGTCCACAAGACTGTCAGCGCTCTGTCCT
  61 ---------+---------+---------+---------+---------+---------+ 120
      AGTTCAAGTTCCTGGCGTACAGGTAGCGGAGACAGGTGTTCTGACAGTCGCGAGACAGGA

      CCAACGCCGACCGCGTCTACAACCACATTTTCCGAGGCTTCGCCGCAACCTCCAATGCCA
 121 ---------+---------+---------+---------+---------+---------+ 180
      GGTTGCGGCTGGCGCAGATGTTGGTGTAAAAGGCTCCGAAGCGGCGTTGGAGGTTACGGT

      ACGACCTCAAGACCCTGCGCGACCACCCTGATGTCGACTACATTGACCACCATGCCATAA
 181 ---------+---------+---------+---------+---------+---------+ 240
      TGCTGGAGTTCTGGGACGCGCTGGTGGGACTACAGCTGATGTAACTGGTGGTACGGTATT

      TCACCATCAAGCGGTACACTCAGCAGCCCGGCGCCCCCTGGGGTCTCGGACGCATCTCTC
 241 ---------+---------+---------+---------+---------+---------+ 300
      AGTGGTAGTTCGCCATGTGAGTCGTCGGGCCGCGGGGGACCCCAGAGCCTGCGTAGAGAG

      ACCGCAGCAAGCGTAGCACCACATACGAGTATGATACCAGCGGCGGCAGTGGCACCTGCG
 301 ---------+---------+---------+---------+---------+---------+ 360
      TGGCGTCGTTCGCATCGTGGTGTATGCTCATACTATGGTCGCCGCCGTCACCGTGGACGC

      CCTATGTCATCGACACTGCCGTCGAGGCTTGGCAGCCCGAGTTCCAGGGCCGCGCCAGCC
 361 ---------+---------+---------+---------+---------+---------+ 420
      GGATACAGTAGCTGTGACGGCAGCTCCGAACCGTCGGGCTCAAGGTCCCGGCGCGGTCGG

      ACCAGATCAAGAGCTTCATCAGCGGCCAGAACACCGACGGCAACGGCCATGGCACTCACT
 421 ---------+---------+---------+---------+---------+---------+ 480
      TGGTCTAGTTCTCGAAGTAGTCGCCGGTCTTGTGGCTGCCGTTGCCGGTACCGTGAGTGA

      GCGCCGGCACCATCGCCTCCAAGACGTACGGTGTTGCCAAGAAGACCAAGATCTACGGTG
 481 ---------+---------+---------+---------+---------+---------+ 540
      CGCGGCCGTGGTAGCGGAGGTTCTGCATGCCACAACGGTTCTTCTGGTTCTAGATGCCAC

      TCAAGGTCCTCGACAACTCGGCCTCCGCTCATACTCGGGCATCATTTCTCCGGTATGGAC
 541 ---------+---------+---------+---------+---------+---------+ 600
      AGTTCCAGGAGCTGTTGAGCCGGAGGCGAGTATGAGCCCGTAGTAAAGAGGCCATACCTG

      TTTGCCGTTCAGGACTCCAAGATCGCGCAGCTGCCCCAAGGGTGTCGTCGCCAATATGTC
 601 ---------+---------+---------+---------+---------+---------+ 660
      AAACGGCAAGTCCTGAGGTTCTAGCGCGTCGACGGGGTTCCCACAGCAGCGGTTATACAG

      TCTGGGCGGTGGAAAGGCTCAGTCTCAAGGACGGTGCCGCTGCCATGATGAGGCGCGCGT
 661 ---------+---------+---------+---------+---------+---------+ 720
      AGACCCGCCACCTTTCCGAGTCAGAGTTCCTGCCACGGCGACGGTACTACTCCGCGCGCA

      CTTCCTCCCCGTCCCGCTGGCAACGACAACGTAACCCCGCCAACTACTCCCCTGCCTCTG
 721 ---------+---------+---------+---------+---------+---------+ 780
      GAAGGAGGGGCAGGGCGACCGTTGCTGTTGCATTGGGGCGGTTGATGAGGGGACGGAGAC

      AGCCGACTGTTTGCACCCTCGGCGCCACCACCTCTTCTGATCCGCGATCTTCGTTCTCCA
 781 ---------+---------+---------+---------+---------+---------+ 840
      TCGGCTGACAAACGTGGGAGCCGCGGTGGTGGAGAAGACTAGGCGCTAGAAGCAAGAGGT

      ACTACGGCAATCTCCTCGACATCTTCGCCCCGGCTAGCAACATTCTGTCCACCTGGTTCG
 841 ---------+---------+---------+---------+---------+---------+ 900
      TGATGCCGTTAGAGGAGCTGTAGAAGCGGGGCCGATCGTTGTAAGACAGGTGGACCAAGC

      GTGGCACTACCAACACCATCTCTGGTACTTCCATGGCCACTCCCCACATTGTTGGTCTCG
 901 ---------+---------+---------+---------+---------+---------+ 960
      CACCGTGATGGTTGTGGTAGAGACCATGAAGGTACCGGTGAGGGGTGTAACAACCAGAGC

      GCGCCTACCTCGCCGCTCTGCACGGTTTCCCCGGCGCCCAGGCGCTCTGCAAGCGCATCC
 961 ---------+---------+---------+---------+---------+---------+ 1020
      CGCGGATGGAGCGGCGAGACGTGCCAAAGGGGCCGCGGGTCCGCGAGACGTTCGCGTAGG

      AGACCCTTTCTTACTAAGAACGTCCTCACCGCATTCCCAGCGGCACTTGTCAACTACCTT
1021 ---------+---------+---------+---------+---------+---------+ 1080
      TCTGGGAAAGAATGATTCTTGCAGGAGTGGCGTAAGGGTCGCCGTGAACAGTTGATGGAA

      CGCCTTCAACGGCAAACCCCAGCGGCTAAATGCTACAAGAATGAGGACTCCTCGACGACC
1081 ---------+---------+---------+---------+---------+---------+ 1140
      GCGGAAGTTGCCGTTTGGGGTCGCCGATTTACGATGTTCTTACTCCTGAGGAGCTGCTGG

      TTTATCCCAGCCATGACCTTTTTTGGAGCATATCAACAGCGGCGAGATCCTCTTGGGACA
1141 ---------+---------+---------+---------+---------+---------+ 1200
      AAATAGGGTCGGTACTGGAAAAAACCTCGTATAGTTGTCGCCGCTCTAGGAGAACCCTGT

      AACCTAAGTATGTGGCATTGAACTGCGCTCCTGTCATATACTCCGCGCAAGGAAGAGTAA
1201 ---------+---------+---------+---------+---------+---------+ 1260
      TTGGATTCATACACCGTAACTTGACGCGAGGACAGTATATGAGGCGCGTTCCTTCTCATT

      AAAGTGGACATGTCTTTTAAATAGCACCAATACATATCTTATGAGCCCTCGAAAAAAAAA
1261 ---------+---------+---------+---------+---------+---------+ 1320
      TTTCACCTGTACAGAAAATTTATCGTGGTTATGTATAGAATACTCGGGAGCTTTTTTTTT

      AAAAAAAAAAAAAAAA
1321 ---------+------ 1336
      TTTTTTTTTTTTTTTT
```

Figure 10

European Patent Office

# EUROPEAN SEARCH REPORT

Application Number

EP 93 10 7233

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-9 102 051 (THE AUSTRALIAN TECHNOLOGICAL INNOVATION CORPORATION)<br>* abstract *<br>--- | 1,6 | C12N9/58<br>A01N63/04<br>//(C12N9/58, C12R1:79) |
| A | US-A-3 515 641 (WHITAKER)<br>* abstract *<br>--- | 6 | |
| A | WO-A-8 803 948 (NOVO INDUSTRI A/S)<br>* abstract *<br>--- | 1,5 | |
| A | WO-A-8 807 581 (AMGEN INC)<br>* abstract *<br>--- | 1,7 | |
| D,A | PHILIPPINE AGRICULTURE<br>vol. 68, 1985, LOS BANOS, LAGUNO<br>pages 159 - 167<br>DAVIDE ET AL. 'Biological control of root-knot nematodes on cotton through the use of fungi Paecilomyces lilacinus .....'<br>----- | | |
| | | | TECHNICAL FIELDS SEARCHED (Int. Cl.5) |
| | | | C12N<br>C12R<br>A01N |

The present search report has been drawn up for all claims

| Place of search | Date of completion of the search | Examiner |
|---|---|---|
| BERLIN | 22 DECEMBER 1993 | CEDER O. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document